# EUROPÄISCHE PATENTSCHRIFT

(11) **EP 3 946 118 B1**
(45) Veröffentlichungstag und Bekanntmachung des Hinweises auf die Patenterteilung: **28.10.2026**
(21) Anmeldenummer: 20718545.5
(22) Anmeldetag: 29.03.2020
(51) Int. Cl.: A61B 18/14, A61B 18/00, A61B 18/12

(54) **BIPOLARES ELEKTROCHIRURGISCHES WERKZEUG**
BIPOLAR ELECTROSURGICAL TOOL
OUTIL ÉLECTROCHIRURGICAL BIPOLAIRE

(30) Priorität: 28.03.2019 DE 102019108141
(43) Veröffentlichungstag der Anmeldung: 09.02.2022
(73) Patentinhaber: Karl Storz SE & Co. KG, 78532 Tuttlingen (DE)
(72) Erfinder: WAGNER, Sebastian, 78532 Tuttlingen (DE); WENZLER, Sebastian, 78532 Tuttlingen (DE); IRION, Klaus, 78532 Tuttlingen (DE); STICKEL, Axel, 78532 Tuttlingen (DE); HINDING, Thomas, 78532 Tuttlingen (DE); HAAF, Emma, 78532 Tuttlingen (DE)
(74) Vertreter: Wimmer, Stephan
(86) Internationale Anmeldenummer: PCT/DE2020/100255
(87) Internationale Veröffentlichungsnummer: WO 2020/192847

(56) Entgegenhaltungen:
- EP-A1- 1 632 192
- EP-A1- 3 308 731
- WO-A2-2017/100423
- WO-A2-2017/100427
- DE-A1- 102006 027 150
- DE-A1- 102011 075 781
- US-A1- 2012 283 735

## Beschreibung

Die vorliegende Erfindung ist auf ein bipolares elektrochirurgisches Werkzeug und auf ein bipolares elektrochirurgisches Instrument bezogen.

Die Vermeidung von Blutungen ist bei allen chirurgischen Verfahren wichtig. Bei mikroinvasiven Eingriffen bergen Blutungen besondere Risiken, da sie die Sicht des operierenden Personals beeinträchtigen können und das Stillen einer Blutung schwieriger sein kann als in der offenen Chirurgie. Der sichere Verschluss von Blutgefäßen - beispielsweise vor deren Durchtrennung - ist deshalb permanent Gegenstand von Forschung und Entwicklung in der Medizintechnik. Ähnliches gilt für den sicheren Verschluss anderer Hohlorgane.

In der Elektrochirurgie erwärmen elektrische Ströme Gewebe, um dieses zu koagulieren. Dies wird oft auch als Verschweißen oder Verschmelzen bezeichnet. Dabei gehen insbesondere kolloidale Stoffe aus einem Lösungszustand in einen Gelzustand über. Bei einer Erwärmung auf eine zu hohe Temperatur kann das betroffene Gewebe jedoch so weit zerstört werden, dass ein sicherer und dauerhafter Verschluss und eine schnelle Heilung nicht mehr gewährleistet sind.

Der Erfolg einer elektrochirurgischen Maßnahme ist von der Art und dem Zustand des Gewebes, von der Stromdichte und ihrer Ortsabhängigkeit, von der Dauer des Stromflusses, der erzielten Temperatur und deren Ortsabhängigkeit, dem mechanischen Druck und weiteren Parametern abhängig.

Ziele bei der Weiterentwicklung elektrochirurgischer Werkzeuge und Instrumente sind ein möglichst genau steuerbarer oder geregelter Prozess mit einer Erwärmung definierter Bereiche zu möglichst definierten Temperaturen und damit eine möglichst vorhersagbare Qualität des Gefäßverschlusses oder allgemeiner der Gewebeveränderung und die Vermeidung des Anhaftens von Gewebe am Werkzeug oder Instrument. Ferner sollen elektrochirurgische Werkzeuge möglichst klein, aber auch mechanisch robust und aufgrund ihrer Gestalt auch zur Präparation geeignet sein.

In EP 1 632 192 A1 ist ein Instrument zum Verschließen von Gefäßen beschrieben, das zwei relativ zu einander bewegbare Greifbacken aufweist (Absätze [0011], [0018]). Jede Greifbacke umfasst ein Paar elektrisch leitfähiger, von einander beabstandeter Gefäßverschlussflächen, die sich längs der Greifbacke erstrecken (ebd.). Jedes Paar Gefäßverschlussflächen ist mit einer Quelle elektrochirurgischer Energie verbunden (ebd.). Die Greifbacken können spiegelbildlich oder nicht spiegelbildlich ausgestaltet sein (Absätze [0059], [0060], [0066], [0067]).

In DE 10 2006 027 150 A1 ist ein elektrochirurgisches Instrument zum Schneiden, Koagulieren und Greifen von Gewebe beschrieben. An den Außenseiten von Greifarmen sind Schneiddrähte oder kleinflächige Drahtelektroden als Schneidelektroden vorgesehen, an den Innenseiten der Greifarme sind großflächige Elektroden als Koagulationselektroden vorgesehen.

In US 6,620,161 B2 ist ein elektrochirurgisches Instrument beschrieben. Ein Schließrohr aus rostfreiem Stahl ist nach distal bewegbar, um ein bewegliches oberes Maulteil zu einem feststehenden unteren Maulteil hin zu bewegen, und nach proximal bewegbar, um das obere Maulteil von dem unteren Maulteil weg zu bewegen. Die Maulteile sind aus einem Kunststoff gebildet. Zwischen den Maulteilen ist ein Schneidelement mit Schneiden in einem unteren Kanal in dem unteren Maulteil und einem oberen Kanal in dem oberen Maulteil in Längsrichtung bewegbar.

In DE 11 2009 000 591 B4 ist eine Zangenbackenanordnung einer bipolaren Zangenvorrichtung beschrieben. Ein erstes Backenelement wird von zwei Gewebe berührenden Elementen gebildet, die durch einen Isolator getrennt sind und Versiegelungselemente zum Versiegeln gegen Gewebe bilden. Das Backenelement ist ferner mit einer Schneideelektrode an dem Isolator versehen. Eine Aussparung verläuft vollständig durch das zweite Backenelement, erzeugt darin eine Öffnung und unterteilt das Backenelement in zwei weitere Gewebe berührende Elemente, die Versiegelungselemente bilden.

In US 2012/0283735 A1 ist eine Vorrichtung zur Befestigung oder Verstärkung von Gewebe beschrieben. Die Vorrichtung umfasst an Maulteilen angeordnete, einander zugewandte Gewebekontaktflächen.

In WO 2017/100427 A2 ist ein Endeffektor mit einer nichtleitenden Klinge und einem Klemmarm beschrieben. An den Seiten des Klemmarms sind zwei Elektroden entgegengesetzter Polarität längs einem Isolator angeordnet und durch diesen isoliert. Ein elektrochirurgischer Hochfrequenz-Pfad erstreckt sich zwischen den Elektroden durch Gewebe.

In WO 2017/100423 A2 ist eine monopolare und eine bipolare Ausführung eines elektrochirurgischen Endeffektors erwähnt. Ferner ist ein Endeffektor beschrieben, der in einem Klemmarm zwei gleich oder entgegengesetzte Elektroden aufweist.

In DE 10 2011 075 781 A1 ist eine Elektrode an einem um seine Längsachse rotierbaren Maulteil beschrieben, die nicht verwendet wird, wenn sie vom gegenüberliegenden Maulteil abgewandt ist.

In EP 3 308 731 A1 ist ein bipolar und monopolar verwendbares elektrochirurgisches Instrument beschrieben. Eine Elektrode an einer von einem zweiten Arm abgewandten Seite eines ersten Arms ist für eine Verwendung in einem monopolaren Modus vorgesehen.

Eine Aufgabe der vorliegenden Erfindung besteht darin, ein verbessertes bipolares elektrochirurgisches Werkzeug und ein verbessertes bipolares elektrochirurgisches Instrument zu schaffen.

Diese Aufgabe wird durch den Gegenstand des unabhängigen Anspruchs gelöst.

Die Erfindung wird im Anspruch 1 definiert. Weiterbildungen sind in den abhängigen Ansprüchen angegeben.

Nachfolgend werden Ausführungsformen beschrieben. Allerdings sind nur die Ausführungsformen, die alle Merkmale des Anspruchs 1 aufweisen, Teil der beanspruchten Erfindung. Die übrigen Ausführungsformen sind nur zu illustrativen Zwecken dargestellt.

Ein bipolares elektrochirurgisches Werkzeug für ein bipolares elektrochirurgisches Instrument umfasst ein erstes Maulteil mit einer ersten Greiffläche, ein zweites Maulteil mit einer der ersten Greiffläche zugewandten zweiten Greiffläche, ein Gelenk, das eine Schwenkbewegung des zweiten Maulteils relativ zu dem ersten Maulteil ermöglicht, eine erste Elektrode an dem ersten Maulteil und eine zweite Elektrode an dem ersten Maulteil, die von der ersten Elektrode elektrisch isoliert ist.

Ein bipolares elektrochirurgisches Werkzeug für ein bipolares elektrochirurgisches Instrument umfasst ein erstes Maulteil, ein zweites Maulteil, ein Gelenk, das eine Schwenkbewegung des zweiten Maulteils relativ zu dem ersten Maulteil ermöglicht, eine erste Greiffläche an dem ersten Maulteil, die dem zweiten Maulteil zugewandt ist, eine zweite Greiffläche an dem zweiten Maulteil, die dem ersten Maulteil zugewandt ist, eine erste Elektrode an dem ersten Maulteil und eine zweite Elektrode, die von der ersten Elektrode elektrisch isoliert ist, wobei die erste Elektrode keiner Elektrode an dem zweiten Maulteil gegenüberliegt.

Bei einem bipolaren elektrochirurgischen Werkzeug, wie es hier beschrieben ist, ist entweder die zweite Elektrode an dem ersten Maulteil angeordnet und liegt keiner Elektrode an dem zweiten Maulteil gegenüber oder die zweite Elektrode ist an dem zweiten Maulteil angeordnet und liegt keiner Elektrode an dem ersten Maulteil gegenüber.

Ein bipolares elektrochirurgisches Werkzeug für ein bipolares elektrochirurgisches Instrument umfasst ein erstes Maulteil mit einer ersten Greiffläche, ein zweites Maulteil mit einer der ersten Greiffläche zugewandten zweiten Greiffläche, ein Gelenk, das eine Schwenkbewegung des zweiten Maulteils relativ zu dem ersten Maulteil ermöglicht, eine erste Elektrode an dem ersten Maulteil und eine zweite Elektroden an dem ersten Maulteil oder an dem zweiten Maulteil, die von der ersten Elektrode elektrisch isoliert ist, wobei die zweite Greiffläche elektrisch isolierend ist soweit sie der ersten Elektrode gegenüberliegt.

Anders ausgedrückt liegt der ersten Elektrode nicht - auch nicht teilweise oder überlappend - ein leitfähiger Bereich oder Teilbereich der zweiten Greiffläche gegenüber. Die Anordnung gegenüber ist besonders auf eine geschlossene Konfiguration des bipolaren elektrochirurgischen Werkzeugs bezogen, bei der die Greifflächen an den Maulteilen den geringsten möglichen Abstand aufweisen und einander in geringem Abstand gegenüberliegen oder teilweise oder vollständig berühren. Ein erster Punkt an einer ersten Fläche liegt einem zweiten Punkt an einer zweiten Fläche gegenüber, wenn er auf einer Flächennormalen der zweiten Fläche in dem zweiten Punkt liegt. Im Fall einer oder zweier gekrümmter Greifflächen wird als Bezugsfläche, auf die die Flächennormale bezogen wird, insbesondere eine ebene Fläche verwendet, welche parallel zu der durch das Gelenk definierten Schwenkachse und parallel zu den Haupterstreckungsrichtungen der Maulteile ist.

Ein bipolares elektrochirurgisches Werkzeug, wie es hier beschrieben ist, ist insbesondere zum Verschließen eines Hohlorgans oder zum Versiegeln von Gewebe vorgesehen und ausgebildet. Das Verschließen eines Hohlorgans oder Versiegeln anderen Gewebes einerseits und das Durchtrennen oder Schneiden eines Hohlorgans andererseits erfordern unterschiedliche Eigenschaften, insbesondere unterschiedliche Geometrien eines bipolaren elektrochirurgischen Werkzeugs. Es mag sein, dass unter bestimmten Bedingungen auch mit einem für das Schneiden vorgesehenen und ausgebildeten bipolaren elektrochirurgischen Werkzeug ein Hohlorgan verschlossen oder anderes Gewebe versiegelt werden kann und unter bestimmten Bedingungen auch mit einem für das Verschließen eines Hohlorgans oder Versiegeln von anderem Gewebe vorgesehenen und ausgebildeten bipolaren elektrochirurgischen Werkzeug ein Hohlorgan durchtrennt werden kann. Die in der Medizin im Sinne der Sicherheit der Patienten unbedingt erforderliche Zuverlässigkeit ist aber nur erzielbar, wenn jedes Werkzeug ausschließlich für den Zweck verwendet wird, für den es vorgesehen und ausgebildet ist. Medizinisches Personal, das ein für das Verschließen eines Hohlorgans oder Versiegeln anderen Gewebes ausgebildetes Werkzeug zum Durchtrennen von Gewebe verwendet oder umgekehrt, handelt außerhalb der medizingeräterechtlichen Zulassung und gefährdet dabei nicht nur die Gesundheit des Patienten, sondern setzt auch sich selbst einem erheblichen Haftungsrisiko aus.

Bei einem bipolaren elektrochirurgischen Werkzeug, wie es hier beschrieben ist, ist entweder die zweite Elektrode an dem ersten Maulteil angeordnet und die zweite Greiffläche elektrisch isolierend soweit sie der zweiten Elektrode gegenüberliegt; oder die zweite Elektrode an dem zweiten Maulteil angeordnet und die erste Greiffläche elektrisch isolierend soweit sie der zweiten Elektrode gegenüberliegt.

Entweder ist die erste Elektrode an dem ersten Maulteil angeordnet, und der ersten Elektrode liegt nicht - auch nicht teilweise oder überlappend - ein leitfähiger Bereich oder Teilbereich der zweiten Greiffläche gegenüber. Oder die erste Elektrode ist an dem zweiten Maulteil angeordnet, und der ersten Elektrode liegt nicht - auch nicht teilweise oder überlappend - ein leitfähiger Bereich oder Teilbereich der ersten Greiffläche gegenüber.

Ein bipolares elektrochirurgisches Werkzeug für ein bipolares elektrochirurgisches Instrument umfasst ein erstes Maulteil, ein zweites Maulteil, ein Gelenk das eine Schwenkbewegung des zweiten Maulteils relativ zu dem ersten Maulteil ermöglicht, eine erste Greiffläche an dem ersten Maulteil, die dem zweiten Maulteil zugewandt ist, eine von dem zweiten Maulteil abgewandten erste Außenfläche an dem ersten Maulteil, eine zweite Greiffläche an dem zweiten Maulteil, die dem ersten Maulteil zugewandt ist, eine von dem ersten Maulteil abgewandte zweite Außenfläche an dem zweiten Maulteil, eine erste Elektrode, die vollständig an der Außenfläche des ersten Maulteils angeordnet ist, und eine zweite Elektrode, die von der ersten Elektrode elektrisch isoliert ist.

Bei einem bipolaren elektrochirurgischen Werkzeug, wie es hier beschrieben ist, ist die zweite Elektrode insbesondere vollständig an der Außenfläche des zweiten Maulteils angeordnet.

Bei einem bipolaren elektrochirurgischen Werkzeug, wie es hier beschrieben ist, ist die erste Greiffläche an dem ersten Maulteil insbesondere vollständig elektrisch isolierend ausgebildet.

Bei einem bipolaren elektrochirurgischen Werkzeug, wie es hier beschrieben ist, ist die zweite Greiffläche an dem zweiten Maulteil insbesondere vollständig elektrisch isolierend ausgebildet.

Bei einem bipolaren elektrochirurgischen Werkzeug, wie es hier beschrieben ist, ist insbesondere der gesamte dem ersten Maulteil zugewandte Oberflächenbereich des zweiten Maulteils elektrisch isolierend ausgebildet, wobei der gesamte dem zweiten Maulteil zugewandte Oberflächenbereich des ersten Maulteils elektrisch isolierend ausgebildet ist.

Ein bipolares elektrochirurgisches Werkzeug für ein bipolares elektrochirurgisches Instrument umfasst ein erstes Maulteil mit einer ersten Greiffläche, ein zweites Maulteil mit einer der ersten Greiffläche zugewandten zweiten Greiffläche, ein Gelenk, das eine Schwenkbewegung des zweiten Maulteils relativ zu dem ersten Maulteil ermöglicht, eine erste Elektrode an dem ersten Maulteil und eine zweite Elektrode an dem ersten Maulteil, die von der ersten Elektrode elektrisch isoliert ist, wobei an dem zweiten Maulteil keine Elektrode angeordnet ist.

Das bipolare elektrochirurgische Instrument ist insbesondere Teil eines mikroinvasiven Instruments (beispielsweise für die Laparoskopie) oder vorgesehen und ausgebildet, um mit einer oder mehreren anderen Komponenten zusammen ein mikroinvasives oder ein anderes elektrochirurgisches Instrument zu bilden. Das bipolare elektrochirurgische Werkzeug ist beispielsweise mit einem distalen Ende eines Schafts eines Instruments dauerhaft mechanisch verbunden, das heißt, nicht ohne Werkzeug oder nicht mit den medizinischem Personal zur Verfügung stehenden Mitteln von dem Schaft zerstörungsfrei und reversibel trennbar. Alternativ kann das bipolare elektrochirurgische Instrument mit einem distalen Ende eines Schafts eines Instruments lösbar verbindbar sein, das heißt, mit den medizinischem Personal zur Verfügung stehenden Mitteln zerstörungsfrei lösbar und wieder verbindbar sein. In beiden Fällen ist insbesondere ein Teil des bipolaren elektrochirurgischen Werkzeugs mechanisch starr mit dem distalen Ende des Schafts verbunden oder verbindbar.

Die erste Greiffläche an dem ersten Maulteil wird insbesondere durch den gesamten dem zweiten Maulteil zugewandten Oberflächenbereich des ersten Maulteils gebildet. Die zweite Greiffläche an dem zweiten Maulteil wird insbesondere durch den gesamten dem ersten Maulteil zugewandten Oberflächenbereich des zweiten Maulteils gebildet. Beide Greifflächen können glatt oder im Wesentlichen glatt, eben oder gekrümmt sein, eine das sichere Greifen und Halten vereinfachende Profilierung und/oder Nuten, Stege, konkave oder konvexe Bereiche aufweisen. Das bipolare elektrochirurgische Werkzeug ist insbesondere so ausgebildet, dass beide Greifflächen einander punktförmig, linienförmig oder flächig berühren können, wenn kein Gewebe zwischen den Greifflächen angeordnet ist.

Das Gelenk ist insbesondere zwischen dem proximalen Ende des zweiten Maulteils und dem proximalen Ende des ersten Maulteils angeordnet. Das Gelenk definiert eine Schwenkachse, die insbesondere orthogonal oder im Wesentlichen orthogonal zu der Längsachse eines mit dem bipolaren elektrochirurgischen Werkzeug in der vorgesehenen Weise verbundenen Schafts und/oder orthogonal zu den Richtungen maximaler Erstreckung der Maulteile ist. Die durch das Gelenk definierte Schwenkachse ist bei der vorgesehenen Verwendung insbesondere parallel oder im Wesentlichen parallel zu einer Längsrichtung eines mittels des bipolaren elektrochirurgischen Werkzeugs zu verschließenden Hohlorgans und damit beispielsweise zur Richtung des Blutflusses in einem zu verschließenden Blutgefäß.

Die erste Elektrode und die zweite Elektrode an dem ersten Maulteil sind insbesondere jeweils streifenförmig und erstrecken sich nebeneinander, parallel oder im Wesentlichen parallel zu der Längsrichtung des ersten Maulteils. Strom, der zwischen der ersten Elektrode und der zweiten Elektrode durch Gewebe, das zwischen den Maulteilen gehalten ist, fließt, fließt deshalb im Wesentlichen parallel zu den Greifflächen, orthogonal zu den Längsrichtungen der Maulteilen und der Elektroden und damit im Wesentlichen parallel zu einer Längsrichtung eines von dem bipolaren elektrochirurgischen Werkzeug gehaltenen Hohlorgans und beispielsweise zur Blutflussrichtung eines von dem bipolaren elektrochirurgischen Werkzeug gehaltenen Blutgefäßes. Die resultierende Verteilung der Stromdichte in dem von dem bipolaren elektrochirurgischen Werkzeug gehaltenen Gewebe kann eine Konzentration der Erwärmung des Gewebes auf den erwünschten Bereich und eine Verbesserung der erzielten oder erzielbaren Qualität des Verschlusses eines Hohlorgans vereinfachen oder ermöglichen.

Eine Anordnung von Elektroden ausschließlich an dem ersten Maulteil kann Konstruktion und Fertigung des zweiten Maulteils deutlich vereinfachen. Auch Konstruktion und Fertigung des Gelenks können vereinfacht sein, da keine elektrisch leitfähige, jedoch von anderen Komponenten elektrisch isolierte Zuleitung zu einer Elektrode an dem zweiten Maulteil erforderlich ist. Ferner wird das Risiko einer Anhaftung von Gewebe an dem zweiten Maulteil deutlich reduziert, wodurch die Anwendung des bipolaren elektrochirurgischen Werkzeugs einfacher und sicherer werden kann. Ein einfacherer Aufbau des zweiten Maulteils kann ferner eine mechanische steifere Konstruktion und damit die Übertragung größerer Kräfte auf mittels des bipolaren elektrochirurgischen Werkzeugs gehaltenes Gewebe vereinfachen.

Ein bipolares elektrochirurgisches Werkzeug für ein bipolares elektrochirurgisches Instrument umfasst ein erstes Maulteil, ein zweites Maulteil, ein Gelenk, das eine Schwenkbewegung des zweiten Maulteils relativ zu dem ersten Maulteil ermöglicht, eine erste Greiffläche an dem ersten Maulteil, die dem zweiten Maulteil zugewandt ist, eine von dem zweiten Maulteil abgewandte erste Außenfläche an dem ersten Maulteil, eine zweite Greiffläche an dem zweiten Maulteil, die dem ersten Maulteil zugewandt ist, eine von dem ersten Maulteil abgewandten zweiten Außenfläche an dem zweiten Maulteil, eine erste Elektrode, die vollständig an der Außenfläche des ersten Maulteils angeordnet ist, und eine zweite Elektrode, die von der ersten Elektrode elektrisch isoliert ist.

Die erste Greiffläche ist insbesondere vollständig elektrisch isolierend.

Die zweite Elektrode ist insbesondere vollständig an der Außenfläche des zweiten Maulteils angeordnet. Nur ein Teil der zweiten Elektrode ist an der zweiten Greiffläche angeordnet, ein weiterer Teil der zweiten Elektrode ist an der zweiten Außenfläche angeordnet. Alternativ ist kein Teilbereich der zweiten Elektrode an der zweiten Greiffläche angeordnet. Insbesondere ist die zweite Greiffläche vollständig elektrisch isolierend.

Die Anordnung mindestens einer Elektrode an einer Außenseite eines Maulteils erfordert nach dem Greifen von Gewebe - beispielsweise eines Blutgefäßes oder eines anderen Hohlorgans - eine Rotation des Werkzeugs, damit das Gewebe an der Elektrode oder an der gesamten Elektrode anliegt. Ein Vorteil der Anordnung beider Elektroden an den Außenseiten der Maulteile ist, dass die gesamte Breite des Maulteils als Bereich, innerhalb dessen eine homogene Stromdichte und eine entsprechend homogene elektrochirurgische Wirkung erzeugt werden kann, zur Verfügung steht. Deshalb kann beispielsweise mit einem kleineren Werkzeug ein zuverlässiger Verschluss eines Blutgefäßes oder eines anderen Hohlorgans bewirkt werden.

Bei einem bipolaren elektrochirurgischen Werkzeug, wie es hier beschrieben ist, ist insbesondere das erste Maulteil mit einem distalen Ende eines Schafts eines elektrochirurgischen Instruments mechanisch starr verbunden oder starr verbindbar.

Das erste Maulteil ist insbesondere als in sich starre Einheit mit einem proximalen Ende des bipolaren elektrochirurgischen Werkzeugs ausgebildet, wobei das proximale Ende des bipolaren elektrochirurgischen Werkzeugs mit einem distalen Ende eines Schafts mechanisch starr verbunden oder starr verbindbar ist. Bei der vorgesehenen Verwendung des bipolaren elektrochirurgischen Werkzeugs wird also nur das zweite Maulteil relativ zu dem Schaft des Instruments geschwenkt. Das erste Maulteil bleibt relativ zu dem Schaft des Instruments unbewegt in einer den Schaft insbesondere geradlinig verlängernden Position.

Die mechanisch starre Verbindung des ersten Maulteils mit dem Schaft vermeidet eine Zuleitung elektrischer Leistung über ein Gelenk hinweg. Damit können elastische elektrische Leitungen oder Schleifkontakte und die damit einhergehenden Risiken und Nachteile vermieden werden. Zu den vermiedenen Risiken und Nachteilen einer Zuleitung elektrischer Leistung über eine elastische elektrische Leitung oder einen Schleifkontakt zählen Materialermüdung, Abrieb, Korrosion und Kontaktwiderstände.

Ferner kann durch die mechanisch starre Verbindung des ersten Maulteils mit dem distalen Ende eines Schafts eine mechanisch robustere und einfachere Konstruktion ermöglicht werden. Der Entfall eines Gelenks zwischen dem ersten Maulteil und dem Schaft kann durch eine vereinfachte Konstruktion auch eine vollständigere und einfachere Reinigung ermöglichen.

Bei einem bipolaren elektrochirurgischen Werkzeug, wie es hier beschrieben ist, ist insbesondere die gesamte zweite Greiffläche elektrisch isolierend ausgebildet.

Bei einem bipolaren elektrochirurgischen Werkzeug, wie es hier beschrieben ist, ist insbesondere die gesamte Oberfläche des zweiten Maulteils elektrisch isolierend ausgebildet.

Eine elektrisch isolierende Ausgestaltung der gesamten zweiten Greiffläche an dem zweiten Maulteil oder der gesamten äußeren Oberfläche des zweiten Maulteils kann das Risiko einer Ausbildung unerwünschter Strompfade über das zweite Maulteil, die den Erfolg einer elektrochirurgischen Maßnahme gefährden können, vermindern.

Bei einem bipolaren elektrochirurgischen Werkzeug, wie es hier beschrieben ist, sind insbesondere ein oder mehrere elektrisch isolierende Bereiche oder Abschnitte des ersten Maulteils und/oder des zweiten Maulteils insbesondere teilweise oder vollständig aus Keramik, Glas, Kunststoff, einem Elastomer oder beschichtetem, insbesondere elektrisch isolierend beschichtetem Metall gebildet.

Ein bipolares elektrochirurgisches Werkzeug, wie es hier beschrieben ist, umfasst insbesondere ferner eine Nut in der ersten Greiffläche.

Ein bipolares elektrochirurgisches Werkzeug, wie es hier beschrieben ist, umfasst insbesondere ferner eine Nut in der zweiten Greiffläche.

Ein bipolares elektrochirurgisches Werkzeug, wie es hier beschrieben ist, umfasst insbesondere ferner eine Schneideinrichtung, die zwischen dem ersten Maulteil und dem zweiten Maulteil bewegbar ist, zum mechanischen Durchtrennen von zwischen dem ersten Maulteil und dem zweiten Maulteil gehaltenen Gewebe.

Die Schneideinrichtung ist insbesondere in Längsrichtung der Maulteile bewegbar. Die parallel zu der vorgesehenen Bewegungsrichtung sich erstreckenden Ränder der Schneideinrichtung können in eine Nut in der ersten Greiffläche und/oder in eine Nut in der zweiten Greiffläche eingreifen und auf diese Weise mechanisch geführt sein. Eine Schneide an dem distalen Ende der Schneideinrichtung kann gerade und dabei orthogonal zu der vorgesehenen Bewegungsrichtung oder schräg zu dieser angeordnet sein, um ein Durchtrennen von Gewebe zu vereinfachen. Alternativ kann die Schneide gekrümmt oder geschwungen sein, wobei sie insbesondere zumindest teilweise schräg, das heißt nicht orthogonal zu der vorgesehenen Bewegungsrichtung verläuft. Die Schneide der Schneideinrichtung erstreckt sich insbesondere im Wesentlichen von dem ersten Maulteil zu dem zweiten Maulteil.

Ein bipolares elektrochirurgisches Werkzeug, wie es hier beschrieben ist, umfasst insbesondere einen Schneiddraht oder eine Schneiddrahtschlinge als monopolare oder bipolare elektrochirurgische Schneideinrichtung, die zwischen dem ersten Maulteil und dem zweiten Maulteil angeordnet ist, zum elektrochirurgischen Durchtrennen von zwischen dem ersten Maulteil und dem zweiten Maulteil gehaltenen Gewebe.

Der Schneiddraht oder die Schneiddrahtschlinge ist insbesondere relativ zu beiden Maulteilen bewegbar. Der Schneiddraht oder die Schneiddrahtschlinge ist insbesondere in einer Richtung parallel zu der Längsrichtung der Maulteile bewegbar. Alternativ oder zusätzlich kann der Schneiddraht oder die Schneiddrahtschlinge in einer Richtung orthogonal zu der Längsrichtung der geschlossenen Maulteile bewegbar sein.

Beispielsweise ist der Schneiddraht an einem distalen Ende eines Maulteils starr, das heißt nicht in seiner Längsrichtung bewegbar befestigt und nimmt in einer Ruheposition eine bogenförmig, rund oder eckig V- oder U-förmige Gestalt an. Dabei kann der Schneiddraht an einem konkaven Oberflächenbereich des Maulteils anliegen oder in einer Nut in einem konkaven Oberflächenbereich des Maulteils teilweise oder vollständig verborgen sein. Durch mechanischen Zug an einem proximalen Bereich des Schneiddrahts kann der Schneiddraht gespannt und damit aus seiner V- oder U-förmigen Gestalt in eine gerade Gestalt überführt werden bis er an dem gegenüberliegenden Maulteil anliegt oder fast anliegt. Dabei kann er Gewebe zwischen den Maulteilen elektrochirurgisch durchtrennen.

Die elektrochirurgische Schneideinrichtung ist insbesondere als monopolare elektrochirurgische Schneideinrichtung ausgebildet, wobei eine großflächige Neutralelektrode außen am Körper des Patienten den Stromkreis schließt. Alternativ oder zusätzlich kann die erste Elektrode und/oder die zweite Elektrode als Gegenelektrode verwendet werden.

Eine Schneideinrichtung zwischen den Maulteilen kann nach einem elektrochirurgischen Verschließen eines Blutgefäßes oder eines anderen Hohlorgans dessen Durchtrennung ermöglichen. Das Verschließen und Durchtrennen kann deshalb besonders sicher und schnell nacheinander erfolgen.

Bei einem bipolaren elektrochirurgischen Werkzeug, wie es hier beschrieben ist, ist insbesondere zumindest entweder die erste Elektrode oder die zweite Elektrode oder ein elektrisch isolierender Oberflächenbereich des ersten Maulteils oder die zweite Greiffläche des zweiten Maulteils teilweise oder vollständig mit einer die mechanische Anhaftung von Gewebe reduzierenden Beschichtung versehen.

Eine die Anhaftung von Gewebe reduzierende oder verhindernde Beschichtung kann die Handhabung und Anwendung des bipolaren elektrochirurgischen Werkzeugs zuverlässiger und sicherer machen.

Die erste Elektrode und die zweite Elektrode sind insbesondere Oberflächenbereiche an Elektrodenbauteilen, die aus einem Material gebildet sind, dessen Wärmeleitfähigkeit möglichst groß ist, insbesondere größer als 10 Wm⁻¹K⁻¹ (beispielsweise Stahl) oder größer als 100 Wm⁻¹K⁻¹ (beispielsweise Silber, Gold oder elektrisch leitfähig dotierter oder beschichteter Diamant).

Eine gute Wärmeabfuhr von der Elektrode durch ein hoch wärmeleitfähiges Elektrodenbauteil kann einer Erwärmung von an der Elektrode anliegendem Gewebe und einer dadurch hervorgerufenen Anhaftung des Gewebes an der Elektrode entgegenwirken.

Bei einem bipolaren elektrochirurgischen Werkzeug, wie es hier beschrieben ist, sind die erste Elektrode und die zweite Elektrode insbesondere nebeneinander und parallel zueinander an der ersten Greiffläche angeordnet.

Die erste Elektrode und die zweite Elektrode sind jeweils insbesondere im Wesentlichen streifenförmig oder schmal rechteckig und erstrecken sich parallel zu der Haupterstreckungsrichtung des ersten Maulteils. Die Anordnung beider Elektroden nebeneinander und parallel zueinander kann eine Stromflussrichtung im Gewebe eines elektrochirurgischen zu verschließenden Hohlorgans parallel zu der ersten Greiffläche und orthogonal zum Querschnitt des Hohlorgans ermöglichen. Die so erzielbaren Stromdichteverteilungen können ein besonders schnelles und besonders sicheres Verschließen des Hohlorgans ermöglichen, insbesondere ohne ein Anhaften von Gewebe an den Elektroden.

Bei einem bipolaren elektrochirurgischen Werkzeug, wie es hier beschrieben ist, sind die erste Elektrode und die zweite Elektrode insbesondere an oder nahe von einander abgewandten Längsrändern der ersten Greiffläche angeordnet sind, wobei die erste Greiffläche zwischen der ersten Elektrode und der zweiten Elektrode vollständig elektrisch isolierend ausgebildet ist.

Die Längsränder sind diejenigen Randabschnitte der Greiffläche, die sich parallel zu einander und zu der Haupterstreckungsrichtung des ersten Maulteils und damit orthogonal zu der Schwenkachse erstrecken. Die Elektroden sind entweder unmittelbar an den Längsrändern der Greiffläche angeordnet, bilden also die Längsränder der Greiffläche, oder weisen Abstände von den Längsrändern der ersten Greiffläche auf, die kleiner oder wesentlich kleiner (kleiner als eine Hälfte oder ein Drittel oder ein Fünftel oder ein Zehntel) als die Breiten der Elektroden sind.

Die Anordnung der Elektroden an oder nahe den Längsrändern der ersten Greiffläche und die vollständig elektrisch isolierende Ausgestaltung der Greiffläche zwischen den Elektroden ermöglicht einen großen Abstand zwischen den Elektroden, so dass der das Gewebe durchfließende und erwärmende Strom über eine vergleichsweise große Distanz im Gewebe fließen kann. Damit kann ein Hohlorgan in einem vergleichsweise breiten Streifen verschlossen oder anderes Gewebe in einem vergleichsweise breiten Streifen versiegelt werden.

Bei einem bipolaren elektrochirurgischen Werkzeug, wie es hier beschrieben ist, ist bei der vorgesehenen Verwendung der Flächeninhalt der ersten Elektrode und der Flächeninhalt der zweiten Elektrode jeweils insbesondere größer als der Querschnitt von in der vorgesehenen Weise zwischen den Maulteilen gegriffenem oder gequetschtem Gewebe während der vorgesehenen elektrochirurgischen Behandlung.

Bei einem bipolaren elektrochirurgischen Werkzeug, wie es hier beschrieben ist, ist bei der vorgesehenen Verwendung die Breite der streifenförmigen ersten Elektrode und die Breite der streifenförmigen zweiten Elektrode insbesondere jeweils größer als die Dicke von in der vorgesehenen Weise zwischen den Maulteilen gegriffenem oder gequetschtem Gewebe während der vorgesehenen elektrochirurgischen Behandlung.

Die vorgesehene Verwendung des bipolaren elektrochirurgischen Werkzeugs und die maximale Querschnittsfläche und Dicke von dabei in der vorgesehenen Weise zwischen den Maulteilen gegriffenem und gequetschtem Gewebe sind insbesondere durch das Zulassungsverfahren und die Spezifikation des bipolaren elektrochirurgischen Werkzeugs eindeutig definiert.

Wenn die Flächeninhalte oder Breiten beider Elektroden größer oder deutlich (insbesondere um mindestens 20 % oder 50 % oder um einen Faktor 2, 3, 5, 10 oder 20) größer ist als die Querschnittsfläche oder Dicke des gegriffenen und/oder gequetschten Gewebes, ist die Stromdichte in dem gegriffenen und gequetschten Gewebe um einen entsprechenden Faktor größer als in dem an den Elektroden anliegenden Gewebe. Die Erwärmung und Veränderung des Gewebes kann deshalb weitgehend auf den gegriffenen und gequetschten Bereich beschränkt sein. Indem an den Elektroden anliegendes Gewebe nicht oder allenfalls geringfügig erwärmt und verändert wird, kann auch ein Anhaften von Gewebe an den Elektroden verhindert oder deutlich vermindert werden.

Bei einem bipolaren elektrochirurgischen Werkzeug, wie es hier beschrieben ist, liegt die Breite der streifenförmigen ersten Elektrode und die Breite der streifenförmigen zweiten Elektrode jeweils insbesondere im Bereich von 0,3 mm bis 5 mm.

Bei der vorgesehenen Verwendung eines bipolaren elektrochirurgischen Werkzeugs, wie es hier beschrieben ist, liegt die Dicke von in der vorgesehenen Weise zwischen den Maulteilen gegriffenem oder gequetschtem Gewebe während der vorgesehenen elektrochirurgischen Behandlung insbesondere im Bereich von 0,05 mm bis 0,4 mm.

Ein bipolares elektrochirurgisches Werkzeug, wie es hier beschrieben ist, umfasst insbesondere ferner einen konvexen Oberflächenbereich zwischen der ersten Elektrode und der zweiten Elektrode, der in Richtung zu dem zweiten Maulteil hin vorsteht.

Der konvexe Oberflächenbereich ist beispielsweise stegförmig oder wulstförmig mit einem im Wesentlichen rechteckigen, trapezförmigen oder abgerundeten Querschnitt. Der konvexe Oberflächenbereich ist insbesondere elektrisch isolierend ausgebildet.

Der konvexe Oberflächenbereich kann ein Komprimieren oder Quetschen von Gewebe in einem Bereich zwischen den Elektroden ermöglichen. Dadurch kann die Stromdichte in dem komprimierten oder gequetschten Bereich wesentlich höher als an den Elektroden sein. Dadurch kann die elektrochirurgische Wirkung auf den durch den konvexen Oberflächenbereich komprimierten oder gequetschten Gewebebereich begrenzt und das Anhaften von Gewebe an den Elektroden verhindert oder vermindert sein.

Die Geometrie des konvexes Oberflächenbereichs definiert im Wesentlichen einen Versiegelungsbereich oder Versiegelungsstreifen, in dem durch Kompression des Gewebes die Stromdichte so hoch ist und in der Folge das Gewebe so weit erwärmt wird, dass es verschmilzt oder verschweißt wird oder versiegelt wird. Die Breite des konvexen Oberflächenbereichs oder eines Plateaus des konvexen Oberflächenbereichs definiert im Wesentlichen die Breite dieses Versiegelungsbereichs. In dem häufigen Fall einer mechanischen oder elektrochirurgischen Durchtrennung des Gewebes nach dem Verschmelzen oder Verschweißen oder Versiegeln hat sich eine Breite des Versiegelungsbereichs auf jeder Seite des nachfolgenden Schnitts in der Größenordnung von 1 mm (insbesondere zwischen 0,8 mm und 1,2 mm) bewährt, insbesondere, wenn das Gewebe ein Blutgefäß oder ein anderes Hohlorgan ist, das zuverlässig verschlossen werden soll. Die Gesamtbreite des konvexen Oberflächenbereichs oder von einem Plateau des konvexen Oberflächenbereichs beträgt damit insbesondere zwischen 1,6 mm und 2,4 mm.

Ein bipolares elektrochirurgisches Werkzeug, wie es hier beschrieben ist, umfasst insbesondere ferner einen elastischen Bereich an der ersten Greiffläche zwischen der ersten Elektrode und der zweiten Elektrode.

Bei einem bipolaren elektrochirurgischen Werkzeug, wie es hier beschrieben ist, ist der konvexe Oberflächenbereich insbesondere durch ein elastisches Bauteil gebildet.

Ein elastischer Bereich an der ersten Greiffläche und insbesondere ein elastischer konvexer Oberflächenbereich zwischen den Elektroden können eine elastische Anpassung an mittels des Werkzeugs gegriffenes oder gequetschtes Gewebe ermöglichen. Diese kann eine gleichmäßige Druckverteilung im Gewebe fördern und eine lokale Überlastung des Gewebes verhindern.

Bei einem bipolaren elektrochirurgischen Werkzeug, wie es hier beschrieben ist, ist der konvexe Oberflächenbereich insbesondere durch ein Bauteil gebildet, das Silikon, ein Elastomer oder einen anderen Kunststoff, Keramik, Glas oder beschichtetes, insbesondere elektrisch isolierend beschichtetes Metall aufweist.

Der konvexe Oberflächenbereich ist insbesondere durch ein Bauteil aus Silikon (Silikonkautschuk, Silikonelastomer oder Silikonharz) mit einer Shore A-Härte im Bereich von 60 bis 80 gebildet. Die elastischen Eigenschaften von Silikon sind innerhalb eines weiten Bereichs und gut reproduzierbar einstellbar. Außerdem kann Silikon eine ausreichende elektrische Durchschlagfestigkeit in der Größenordnung von 20 kV/mm aufweisen.

Bei einem bipolaren elektrochirurgischen Werkzeug, wie es hier beschrieben ist, ist der konvexe Oberflächenbereich insbesondere durch ein Bauteil gebildet, das ein Material mit einer geringeren thermischen Leitfähigkeit aufweist.

Die Wärmeleitfähigkeit des den konvexen Oberflächenbereich bildenden Bauteils beträgt deutlich weniger als 1 Wm⁻¹K⁻¹, insbesondere weniger als 0,25 Wm⁻¹K⁻¹ und bevorzugt höchstens 0,2 Wm⁻¹K⁻¹ oder höchstens 0,15 Wm⁻¹K⁻¹ oder höchstens 0,1 Wm⁻¹K⁻¹.

Eine geringe thermische Leitfähigkeit verringert den Abfluss von Wärme aus dem durch den konvexen Oberflächenbereich komprimierten und elektrochirurgisch behandelten Gewebebereich und kann so zu einer Konzentration der elektrochirurgischen Wirkung auf den komprimierten Bereich beitragen.

Bei einem bipolaren elektrochirurgischen Werkzeug, wie es hier beschrieben ist, ist zumindest entweder die erste Elektrode oder die zweite Elektrode von dem konvexen Oberflächenbereich beabstandet.

Eine Beabstandung der Elektroden von dem konvexen Oberflächenbereich kann bewirken, dass das Gewebe im Bereich der Elektroden einen deutlich größeren Querschnitt aufweist als in dem durch den konvexen Oberflächenbereich komprimierten Gebiet. Deshalb können die Stromdichte und die elektrochirurgische Wirkung auf den durch den konvexen Oberflächenbereich komprimierten Bereich konzentriert werden und an den Elektroden deutlich kleiner sein. Dies kann Zuverlässigkeit und Qualität der elektrochirurgischen Maßnahme deutlich verbessern und das Risiko eines Anhaftens von Gewebe an den Elektroden verringern.

Bei einem bipolaren elektrochirurgischen Werkzeug, wie es hier beschrieben ist, umfasst der konvexe Oberflächenbereich insbesondere eine Schneide zum mechanischen Durchtrennen von Gewebe.

Der konvexe Oberflächenbereich und die Schneide sind insbesondere so ausgebildet, dass bei Ausübung einer ersten, geringeren vorbestimmten Zuhaltekraft ein elektrochirurgisches Verschließen eines Hohlorgans und bei einer zweiten, höheren vorbestimmten Zuhaltekraft ein mechanisches Durchtrennen des zuvor verschlossenen Hohlorgans möglich ist.

Bei einem bipolaren elektrochirurgischen Werkzeug, wie es hier beschrieben ist, ist der konvexe Oberflächenbereich insbesondere dachförmig ausgebildet.

Eine dachförmige Ausgestaltung mit einer Kante, die am weitesten zu dem zweiten Maulteil hin vorsteht, und zwei beiderseits der Kante abfallenden Flächen kann ein Quetschen von Gewebe derart ermöglichen, dass dessen Querschnitt oder Dicke beiderseits bis zu der Kante kontinuierlich abnimmt und an der Kante minimal ist. Es resultiert eine Stromdichte, die beiderseits der Kante zu der Kante hin zunimmt. Diese Stromdichte kann eine Temperaturverteilung bewirken, die beiderseits der Kante in vorbestimmten oder von den jeweiligen Bedingungen einer elektrochirurgischen Maßnahme abhängigen Abständen von der Kante eine Koagulation und einen Verschluss eines Hohlorgans und im Bereich der Kante eine so starke Erwärmung bewirken kann, dass im Bereich der Kante das Gewebe durchtrennt wird.

Ein bipolares elektrochirurgisches Werkzeug, wie es hier beschrieben ist, umfasst insbesondere ferner einen konvexen Oberflächenbereich an der zweiten Greiffläche des zweiten Maulteils, der zu der ersten Greiffläche des ersten Maulteils hin vorsteht.

Der konvexe Oberflächenbereich an der zweiten Greiffläche des zweiten Maulteils kann ähnliche oder gleiche Eigenschaften, Merkmale und Funktionen wie der beschriebene konvexe Oberflächenbereich an der ersten Greiffläche des ersten Maulteils aufweisen. Das bipolare elektrochirurgische Werkzeug kann entweder nur an der ersten Greiffläche an dem ersten Maulteil oder nur an der zweiten Greiffläche an dem zweiten Maulteil einen konvexen Oberflächenbereich oder an beiden Greifflächen an beiden Maulteilen jeweils einen konvexen Oberflächenbereich aufweisen. Wenn beide Greifflächen an beiden Maulteilen jeweils einen konvexen Oberflächenbereich aufweisen, können deren Querschnitte spiegelsymmetrisch oder unterschiedlich ausgestaltet sein. Ferner können die konvexen Oberflächenbereiche aus Bauteilen aus gleichen oder unterschiedlichen Materialien gebildet sein.

Bei einem bipolaren elektrochirurgischen Werkzeug, wie es hier beschrieben ist, ist der konvexe Oberflächenbereich an der zweiten Greiffläche des zweiten Maulteils insbesondere so angeordnet und ausgebildet, dass er auch im geschlossenen Zustand des Werkzeugs weder die erste Elektrode noch die zweite Elektrode berühren kann.

Der durch den konvexen Oberflächenbereich an der zweiten Greiffläche des zweiten Maulteils komprimierte oder gequetschte Gewebebereich reicht somit nicht bis zu den Elektroden. Von dem Werkzeug gegriffenes und gequetschtes Gewebe kann deshalb in der Nähe der Elektroden weniger komprimiert als im Bereich des konvexen Oberflächenbereichs. Deshalb sind die Querschnitte des Gewebes im Bereich der Elektroden deutlich größer als bei dem konvexen Oberflächenbereich. Es resultiert eine geringere Stromdichte an den Elektroden und eine höhere Stromdichte in dem durch den konvexen Oberflächenbereich komprimierten Gewebebereich.

Bei einem bipolaren elektrochirurgischen Werkzeug, wie es hier beschrieben ist, ist der konvexe Oberflächenbereich an der zweiten Greiffläche des zweiten Maulteils insbesondere durch ein Bauteil aus einem elastischen Material gebildet.

Ein bipolares elektrochirurgisches Werkzeug, wie es hier beschrieben ist, umfasst insbesondere ferner einen Sensor zur Erfassung einer Temperatur oder eines mechanischen Drucks oder einer Farbe oder einer Lichtintensität von reflektiertem oder transmittiertem Licht oder einer anderen physikalischen Größe, um eine elektrochirurgische Maßnahme oder deren Arbeitsergebnis zu steuern oder zu überwachen oder zu prüfen.

Der Sensor kann zur Erfassung eines Lichtspektrums, beispielsweise eines Spektrums von reflektiertem oder transmittiertem Licht und/oder zur Erfassung von Licht bei einer oder mehreren vorbestimmten Wellenlängen oder in einem oder mehreren schmalen Wellenlängenbereichen vorgesehen und ausgebildet sein. Eine Steuerung, Regelung oder Überwachung einer elektrochirurgischen Maßnahme mit einem Sensor kann die Zuverlässigkeit des Verfahrens und die Reproduzierbarkeit des Arbeitsergebnisses deutlich verbessern.

Ein bipolares elektrochirurgisches Instrument umfasst einen Schaft und ein bipolares elektrochirurgisches Werkzeug, wie es hier beschrieben ist, wobei das bipolare elektrochirurgische Werkzeug mit einem distalen Ende des Schafts verbunden oder verbindbar ist.

Das bipolare elektrochirurgische Werkzeug ist mit dem distalen Ende des Schafts insbesondere mechanisch starr verbunden oder verbindbar. Die mechanische Verbindung zwischen dem bipolaren elektrochirurgischen Werkzeug und dem distalen Ende des Schafts kann dauerhaft, das heißt, mit den medizinischem Personal zur Verfügung stehenden Mitteln nicht zerstörungsfrei lösbar, oder mit den medizinischem Personal zur Verfügung stehenden Mitteln zerstörungsfrei und reversibel lösbar sein.

### Kurzbeschreibung der Figuren

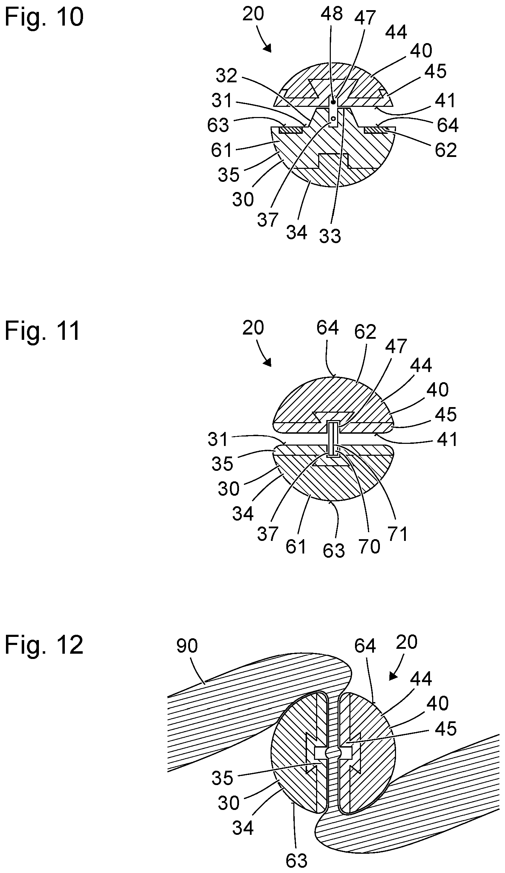

Nachfolgend werden Ausführungsformen anhand der beigefügten Figuren näher erläutert. Allerdings fallen nur die in den Figuren 11 und 12 dargestellten Ausführungsformen unter den beanspruchten Gegenstand.

Es zeigen:
- Figur 1: eine schematische Darstellung eines distalen Endes eines bipolaren elektrochirurgischen Instruments;
- Figur 2: eine weitere schematische Darstellung des distalen Endes des bipolaren elektrochirurgischen Instruments aus Figur 1;
- Figur 3: eine schematische Darstellung eines Querschnitts des bipolaren elektrochirurgischen Werkzeugs des Instruments aus den Figuren 1 und 2
- Figur 4: eine schematische Darstellung eines distalen Endes eines weiteren bipolaren elektrochirurgischen Instruments;
- Figur 5: eine schematische Darstellung eines Querschnitts eines weiteren bipolaren elektrochirurgischen Werkzeugs;
- Figur 6: eine schematische Darstellung eines Querschnitts eines weiteren bipolaren elektrochirurgischen Werkzeugs;
- Figur 7: eine schematische Darstellung eines Werkzeugs eines weiteren bipolaren elektrochirurgischen Instruments;
- Figur 8: eine schematische Darstellung eines Werkzeugs eines weiteren bipolaren elektrochirurgischen Instruments;
- Figur 9: eine weitere schematische Darstellung des Werkzeugs aus Figur 8;
- Figur 10: eine schematische Darstellung eines Querschnitts des Werkzeugs aus den Figuren 8 und 9;
- Figur 11: eine schematische Darstellung eines Querschnitts eines weiteren Werkzeugs;
- Figur 12: eine weitere schematische Darstellung des Querschnitts des Werkzeugs aus Figur 11.

### Beschreibung der Ausführungsformen

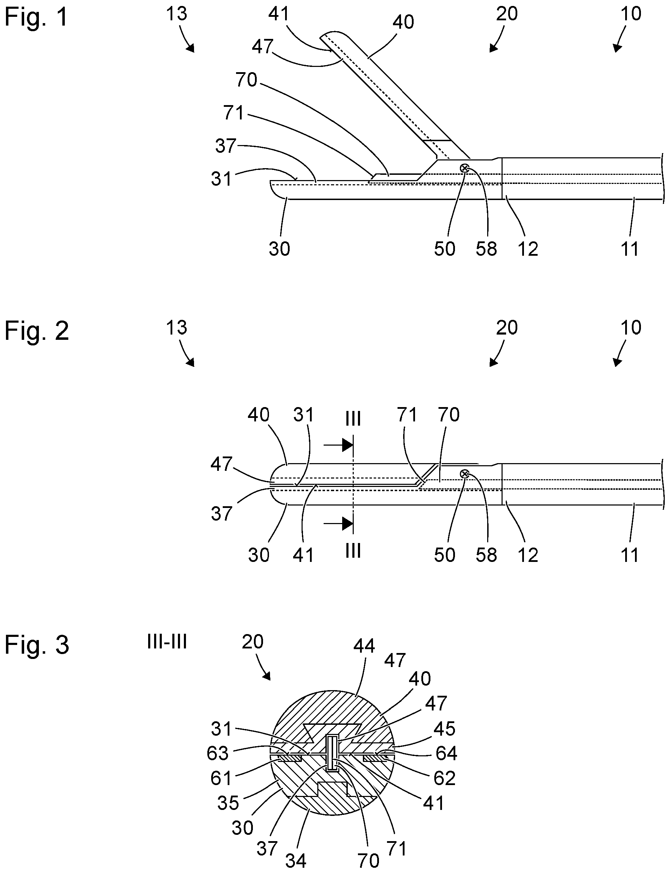

Figur 1 zeigt eine schematische Darstellung eines distalen Endes 13 eines bipolaren elektrochirurgischen Instrument 10. Das bipolare elektrochirurgische Instrument 10 weist einen Schaft 11 auf, der gerade oder gekrümmt, starr oder flexibel ausgebildet sein kann. Ein proximales Ende des Schafts 11 ist mit einer Handhabungseinrichtung verbunden oder verbindbar, die in Figur 1 nicht dargestellt ist. Ein distales Ende 12 des Schafts 11 ist mit einem bipolaren elektrochirurgischen Werkzeug 20 dauerhaft verbunden oder zerstörungsfrei lösbar verbindbar. Das bipolare elektrochirurgische Werkzeug 20 bildet das distale Ende 13 des bipolaren elektrochirurgischen Instruments 10.

Das bipolare elektrochirurgische Werkzeug 20 umfasst eine erste Branche oder ein erstes Maulteil 30 mit einer ersten Greiffläche 31 und eine zweite Branche oder ein zweites Maulteil 40 mit einer zweiten Greiffläche 41. Die erste Greiffläche 31 an dem ersten Maulteil 30 ist dem zweiten Maulteil 40 zugewandt. Die zweite Greiffläche 41 an dem zweiten Maulteil 40 ist dem ersten Maulteil 30 zugewandt. Das proximale Ende des zweiten Maulteils 40 ist über ein Gelenk 50 mit dem übrigen bipolaren elektrochirurgischen Werkzeug 20 schwenkbar verbunden. Das Gelenk 50 definiert eine Schwenkachse 58 orthogonal zu der Längsachse des Schafts 11, orthogonal zu der Zeichenebene der Figur 1 und orthogonal zu den Haupterstreckungsrichtungen der Maulteile 30, 40.

In dem Schaft 11 ist eine in Figur 1 nicht dargestellte Kraftübertragungseinrichtung angeordnet, die sich von dem proximalen Ende des Schafts 11 bis zu dem bipolaren elektrochirurgischen Werkzeug 20 erstreckt. Das distale Ende der Kraftübertragungseinrichtung ist derart mit dem zweiten Maulteil 40 gekoppelt, dass eine an der in Figur 1 nicht dargestellten Handhabungseinrichtung bewirkte Bewegung der Kraftübertragungseinrichtung eine Schwenkbewegung des zweiten Maulteils 40 um die durch das Gelenk 50 definierte Schwenkachse 58 relativ zu dem distalen Ende 12 des Schafts 11 und zu dem ersten Maulteil 30 bewirkt.

In Figur 1 ist das bipolare elektrochirurgische Werkzeug 20 in einer offenen Konfiguration gezeigt. Ausgehend von der in Figur 1 gezeigten offenen Konfiguration kann das zweite Maulteil 40 auf das erste Maulteil 30 zu bewegt werden. Dabei kann Gewebe zwischen den Maulteilen 30, 40 gegriffen, gehalten oder gequetscht werden.

Das bipolare elektrochirurgische Werkzeug 20 umfasst eine Nut 37 in der ersten Greiffläche 31 an dem ersten Maulteil 30 und eine Nut 47 in der zweiten Greiffläche 41 an dem zweiten Maulteil 40. Beide Nuten 37, 47 sind bei der Darstellung in Figur 1 an sich nicht sichtbar. Deshalb sind lediglich die Konturen der Nuten in Figur 1 durch gestrichelte Linien angedeutet.

Das bipolare elektrochirurgische Instrument 10 umfasst ein Skalpell 70, das auch dann, wenn die Maulteile 30, 40 aneinander anliegen, in den Nuten 37, 47 bewegt werden kann. Dabei ist das Skalpell 70 durch die Nuten 37, 47 geführt. Ein aus der Nut 37 in der ersten Greiffläche 31 an dem ersten Maulteil 30 herausragender Teil des Skalpells 70 ist in Figur 1 sichtbar und mit durchgezogener Linie dargestellt. Ein in der Nut 37 in dem ersten Maulteil 30 verborgener Teil des Skalpells 70 ist in Figur 1 in kurz gestrichelter Linie dargestellt.

Das Skalpell 70 ist in Figur 1 in kurz gestrichelten Linien nach proximal fortgesetzt dargestellt, um anzudeuten, dass das Skalpell 70 vom proximalen Ende des Instruments 10 her gesteuert bewegt werden kann. Die mechanische Kopplung zwischen einer Handhabungseinrichtung des Instruments 10 und dem Skalpell 70 kann jedoch nicht nur durch eine unmittelbare Fortsetzung des das Skalpell 70 bildenden Bauteils nach proximal, sondern auch auf andere Weise erfolgen.

Das Skalpell 70 weist eine Schneide 71 auf, die bei dem dargestellten Beispiel relativ zu der vorgesehenen Bewegungsrichtung des Skalpells 70 geneigt ist. In Figur 1 befinden das Skalpell 70 und die Schneide 71 sich in einer Position, in der das Skalpell 70 zwischen geschlossenen Maulteilen 30, 40 gefasstes Gewebe bereits teilweise durchtrennt hätte. In dieser Position ist das Skalpell 70 in Figur 1 besser sichtbar. Bei der vorgesehenen Verwendung des bipolaren elektrochirurgischen Werkzeugs 20 verbleibt das Skalpell 70 jedoch in einer vorbestimmten proximalen Position, in der die Schneide 71 nicht exponiert ist und kein Gewebe berühren kann, solange die Maulteile 30, 40 nicht geschlossen sind.

Figur 2 zeigt eine weitere schematische Darstellung des distalen Endes 13 des anhand der Figur 1 dargestellten bipolaren elektrochirurgischen Instruments 10. Die Art der Darstellung in Figur 2 entspricht derjenigen der Figur 1. In Figur 2 ist das bipolare elektrochirurgische Werkzeug 20 jedoch in einer Konfiguration dargestellt, die von der in Figur 1 dargestellten Konfiguration abweicht.

Bei der in Figur 2 dargestellten Konfiguration des bipolaren elektrochirurgischen Werkzeugs 2 sind die Maulteile 30, 40 geschlossen, die Greifflächen 31, 41 der Maulteile 30, 40 berühren einander oder liegen einander in einem kleinen Abstand gegenüber. Bei der in Figur 2 gezeigten Konfiguration oder Situation ist ferner das Skalpell 70 in der beschriebenen vorbestimmten proximalen Position angeordnet, in der die Schneide 71 nicht exponiert ist.

Bei der in Figur 2 dargestellten Konfiguration bzw. Situation kann Gewebe, beispielsweise ein Blutgefäß oder ein anderes Hohlorgan, zwischen den Maulteilen 30, 40 gefasst und komprimiert oder gequetscht sein. Dieses kann mittels des bipolaren elektrochirurgischen Werkzeugs 20 wie nachfolgend anhand der Figur 3 beschrieben, elektrochirurgisch verändert werden, um beispielsweise das Hohlorgan zu verschließen. Danach kann das Skalpell 70 ausgehend von der in Figur 2 gezeigten Position nach distal bewegt werden, um das Gewebe zu durchtrennen.

Figur 3 zeigt eine schematische und vergrößerte Darstellung eines Schnitts entlang der in Figur 2 angedeuteten Schnittebene III-III durch das in Figur 2 gezeigte bipolare elektrochirurgische Werkzeug 20. Die Schnittebene III-III der Figur 3 ist orthogonal zu den Zeichenebenen der Figuren 1 und 2 und orthogonal zu einer Haupterstreckungsrichtung der Maulteile 30, 40.

In Figur 3 sind die jeweils im Wesentlichen halbkreisförmigen Querschnitte der Maulteile 30, 40 erkennbar. Das erste Maulteil 30 ist aus zwei Bauteilen 34, 35 gebildet, die dauerhaft und starr mechanisch verbunden sind, insbesondere durch Schweißen oder Kleben oder auf eine andere stoffschlüssige Weise. Bei dem dargestellten Beispiel ist das von der ersten Greiffläche 31 beabstandete erste Bauteil 34 aus Metall gebildet und das die Greiffläche 31 bildende zweite Bauteil 35 aus einem elektrisch isolierenden Material.

In dem zweiten Bauteil 35 des ersten Maulteils 30 ist die bereits anhand der Figuren 1 und 2 beschriebene Nut 37 im Querschnitt erkennbar.

Ferner sind an die erste Greiffläche 31 angrenzend zwei Elektrodenbauteile 61, 62 in das elektrisch isolierende zweite Bauteil 35 eingebettet. Freiliegende Oberflächenbereiche der Elektrodenbauteile 61, 62 bilden Elektrodenflächen 63, 64. Die Elektrodenbauteile 61, 62 und die von ihnen gebildeten Elektrodenflächen 63, 64 sind jeweils im Wesentlichen streifenförmig ausgebildet und parallel zueinander und zu der Haupterstreckungsrichtung des ersten Maulteils 30 angeordnet.

Die Elektrodenflächen 63, 64 ermöglichen bei unmittelbarem Kontakt mit Gewebe einen Übertritt von elektrischem Strom von dem ersten Elektrodenbauteil 61 in das Gewebe und von dem Gewebe in das zweite Elektrodenbauteil 62 oder umgekehrt. Zwischen den Elektrodenbauteilen 61, 62 durch Gewebe zwischen den Greifflächen 31, 41 fließender elektrischer Strom fließt im Wesentlichen parallel zu den Greifflächen 31, 41 der Maulteile 30, 40 und orthogonal zu den Haupterstreckungsrichtungen der Maulteile 30, 40.

Die Elektrodenbauteile 61, 62 sind aus einem Material gebildet, dessen Wärmeleitfähigkeit möglichst groß ist, insbesondere größer als 10 Wm⁻¹K⁻¹ (beispielsweise Stahl) oder größer als 100 Wm⁻¹K⁻¹ (beispielsweise Silber, Gold oder elektrisch leitfähig dotierter oder beschichteter Diamant). Eine gute Wärmeleitfähigkeit der Elektrodenbauteile 61, 62 ermöglicht eine gute Wärmeabfuhr von den Elektrodenflächen 63, 64 weg. Deshalb bleiben die Temperaturen der Elektrodenflächen 63, 64 und von an den Elektrodenflächen 63, 64 anliegendem Gewebe niedrig. Dies reduziert die Wahrscheinlichkeit einer Anhaftung von Gewebe an den Elektrodenflächen 63, 64.

Das zweite Maulteil 40 ist bei dem dargestellten Beispiel ebenfalls aus zwei Bauteilen 44, 45 gebildet. Die Bauteile 44, 45 des zweiten Maulteils 40 sind bei dem dargestellten Beispiel formschlüssig, nämlich durch schwalbenschwanzförmige Querschnitte, miteinander mechanisch verbunden. Alternativ oder zusätzlich können die Bauteile 44, 45 des zweiten Maulteils 40 stoffschlüssig miteinander verbunden sein, beispielsweise durch Schweißen oder Kleben.

Die bereits erwähnte Nut 47 in der zweiten Greiffläche 41 an dem zweiten Maulteil 40 ist ausschließlich in dem zweiten Bauteil 45 des zweiten Maulteils 40 gebildet. In Figur 3 ist erkennbar, dass das Skalpell 70 mit der Schneide 71 in den Nuten 37, 47 in den Maulteilen 30, 40 spiel- und reibungsarm geführt ist.

Bei dem dargestellten Beispiel ist das erste Bauteil 44 des zweiten Maulteils 40 aus Metall gebildet, das zweite Bauteil 45 des zweiten Maulteils 40 ist aus einem elektrisch isolierenden Material gebildet. Das zweite Maulteil 40 weist keine Elektroden auf.

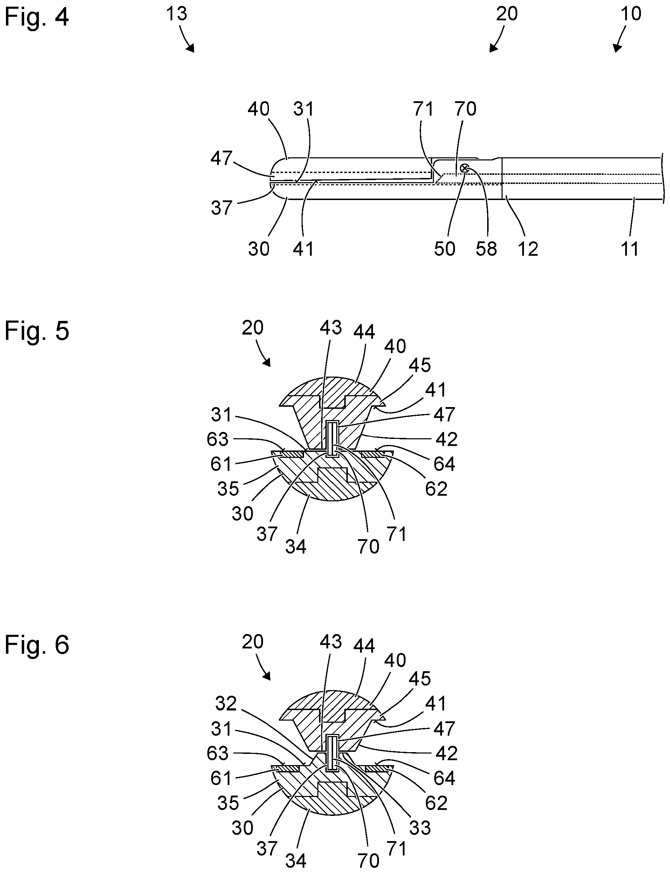

Figur 4 zeigt eine schematische Darstellung eines distalen Endes 13 eines weiteren bipolaren elektrochirurgischen Instruments 10, das in einigen Merkmalen, Eigenschaften und Funktionen dem anhand der Figuren 1 bis 3 dargestellten Instrument ähneln kann. Die Art der Darstellung in Figur 4 entspricht derjenigen der Figuren 1 und 2. Nachfolgend sind insbesondere Merkmale, Eigenschaften und Funktionen des in Figur 4 gezeigten Instruments 10, in denen dieses sich von dem anhand der Figuren 1 bis 3 dargestellten Instrument unterscheidet, beschrieben.

Das in Figur 4 gezeigte Instrument 10 umfasst ein bipolares elektrochirurgisches Werkzeug 20 an seinem distalen Ende 13, das sich von dem anhand der Figuren 1 bis 3 dargestellten bipolaren elektrochirurgischen Werkzeug einerseits durch eine etwas andere Ausgestaltung der Umgebung des Gelenks 50 und andererseits vor allem durch die Anordnung der Greifflächen 31, 41 unterscheidet. Bei der in Figur 4 gezeigten Situation berühren die Greifflächen 31, 41 der Maulteile 30, 40 einander nur am distalen Ende der Maulteile 30, 40. Die Greifflächen 31, 41 bilden einen kleinen Winkel von wenigen Grad. Dies bewirkt, dass, wenn Gewebe zwischen den Greifflächen 31, 41 angeordnet und komprimiert ist, die Dicke dieses Gewebes anders als bei dem Ausführungsbeispiel der Figuren 1 bis 3 von proximal nach distal weniger stark zunimmt oder sogar konstant ist.

Figur 5 zeigt eine schematische Darstellung eines Schnitts durch Maulteile 30, 40 eines weiteren bipolaren elektrochirurgischen Werkzeugs, das in einigen Merkmalen, Eigenschaften und Funktionen den anhand der Figuren 1 bis 4 dargestellten Werkzeugen ähnelt. Die Art der Darstellung, insbesondere die Schnittebene, entspricht derjenigen der Figur 3. Nachfolgend sind insbesondere Merkmale, Eigenschaften und Funktionen des in Figur 5 gezeigten bipolaren elektrochirurgischen Werkzeugs 20 beschrieben, in denen dieses sich von den anhand der Figuren 1 bis 4 dargestellten Werkzeugen unterscheidet.

Der Querschnitt des ersten Maulteils 30 des in Figur 5 gezeigten bipolaren elektrochirurgischen Werkzeugs 20 ähnelt weitgehend dem anhand der Figur 3 dargestellten Querschnitt. Bei dem dargestellten Beispiel ist lediglich der Querschnitt des ersten Maulteils 30 etwas flacher, und die Nut 37 in dem ersten Maulteil 30 ist weniger tief.

Ähnlich wie bei dem anhand der Figur 3 dargestellten Beispiel umfasst das zweite Maulteil 40 ein erstes Bauteil 44 aus einem Metall, das das zweite Maulteil 40 steifer macht. Ähnlich wie bei dem anhand der Figur 3 dargestellten Beispiel ist die gesamte zweite Greiffläche 41 an dem zweiten Maulteil 40 - einschließlich des konvexen Bereichs 42 - durch ein Bauteil 45 aus einem elektrisch isolierenden Material gebildet.

Das zweite Maulteil 40 des in Figur 5 gezeigten bipolaren elektrochirurgischen Werkzeugs 20 unterscheidet sich von dem anhand der Figur 3 dargestellten dadurch, dass die zweite Greiffläche an dem zweiten Maulteil 40 einen konvexen Bereich 42 aufweist, der in Richtung zu dem ersten Maulteil 30 ragt. Dieser konvexe Bereich 42 bildet bei dem dargestellten Beispiel den größten Teil der zweiten Greiffläche 41 an dem zweiten Maulteil.

Das elektrisch isolierende zweite Bauteil 45 des zweiten Maulteils 40 weist einen Querschnitt mit einem trapezförmigen Abschnitt, der den konvexen Bereich 42 der zweiten Greiffläche 41 bildet, auf. Die schrägen Flanken des konvexen Bereichs 42 der zweiten Greiffläche 41, also die beiden nicht parallelen Seiten des trapezförmigen Abschnitts des Querschnitts des elektrisch isolierenden zweiten Bauteils 45, schließen einen Winkel von ca. 20° ein. Der dem ersten Maulteil 30 zugewandte und bei der in Figur 5 gezeigten Konfiguration oder Situation an der ersten Greiffläche 31 anliegende oder diesem in kleinem Abstand gegenüberliegende plateauförmige Teilbereich 43 des konvexen Bereichs 42 der zweiten Greiffläche 41 an dem zweiten Maulteil 40 ist so schmal, dass er auch in der in Figur 5 gezeigten Konfiguration oder Situation nicht mit den Elektrodenflächen 63, 64 überlappt oder diese berührt, sondern von diesen einen Abstand aufweist. Zwischen den Greifflächen 31, 41 der Maulteile 30, 40 angeordnetes und komprimiertes oder gequetschtes Gewebe ist deshalb im Bereich der Elektrodenflächen 63, 64 nicht oder nur geringfügig gequetscht und weist große Querschnitte auf, die geringe Stromdichten zur Folge haben.

Der plateauförmige Teilbereich 43 des konvexen Bereichs 42 ist eben oder im Wesentlichen eben und liegt der ersten Greiffläche 31 parallel oder im Wesentlichen parallel gegenüber. Bei der vorgesehenen Verwendung des Werkzeugs 20 wird Gewebe mit mechanischen Eigenschaften innerhalb eines vorbestimmten Bereichs mittels einer vorgesehenen Schließkraft der Maulteile 30, 40 zwischen den Greifflächen 31, 41 komprimiert. Bei dieser vorgesehenen Verwendung ist Gewebe im Wesentlichen nur zwischen dem plateauförmigen Teilbereich 43 des konvexen Bereichs 42 und der ersten Greiffläche 31 deutlich komprimiert und wird von einer ausreichend hohen Stromdichte durchflossen und ausreichend erwärmt, damit das Gewebe elektrochirurgisch verschmilzt oder verschweißt wird oder versiegelt wird. Abseits des plateauförmigen Bereichs 43 ist das Gewebe nicht oder deutlich weniger komprimiert. Entsprechend sind dort die Stromdichte und die Erwärmung deutlich geringer, und es der Strom hat keine oder im Wesentlichen keine elektrochirurgische Wirkung. Die Breite des plateauförmigen Teilbereichs bestimmt also die Breite des Versiegelungsbereichs oder Versiegelungsstreifens, innerhalb dessen das Gewebe versiegelt wird. Die Breite des plateauförmigen Teilbereichs liegt insbesondere im Bereich von 1,6 mm bis 2,4 mm.

In dem elektrisch isolierenden zweiten Bauteil 45 des zweiten Maulteils 40 ist die bereits beschriebene Nut 47 vorgesehen, in der das Skalpell 70 spiel- und reibungsarm geführt ist. Bei dem dargestellten Beispiel ist die Nut 47 in dem zweiten Bauteil 45 des zweiten Maulteils 40 deutlich tiefer als die Nut 37 in dem zweiten Bauteil 35 des ersten Maulteils 30, sodass das Skalpell 70 überwiegend in dem zweiten Maulteil 40 geführt ist.

Das gesamte elektrisch isolierende zweite Bauteil 45 des zweiten Maulteils 40 oder der konvexe Bereich 42 weist eine geringe Wärmeleitfähigkeit auf, um einen Abfluss von Wärme aus dem Versiegelungsbereich oder Versiegelungsstreifen zu reduzieren und die elektrochirurgische Wirkung in dem Versiegelungsbereich zu erhöhen. Gleiches gilt für das gesamte elektrisch isolierende zweite Bauteil 35 des ersten Maulteils 30 oder dessen Bereich zwischen den Elektrodenbauteilen 61, 62.

Das gesamte elektrisch isolierende zweite Bauteil 45 des zweiten Maulteils 40 oder der konvexe Bereich 42 weist insbesondere Silikonkautschuk, Silikonelastomer oder Silikonharz und eine Shore A-Härte im Bereich von 60 bis 80 auf.

Figur 6 zeigt eine schematische Darstellung eines Schnitts durch Maulteile 30, 40 eines weiteren bipolaren elektrochirurgischen Werkzeugs 20, das in einigen Merkmalen, Eigenschaften und Funktionen den anhand der Figuren 1 bis 5 dargestellten Werkzeugen ähnelt. Die Art der Darstellung, insbesondere die Position und Orientierung der in Figur 6 gezeigten Schnittebene entspricht derjenigen der Figuren 3 und 5. Nachfolgend sind insbesondere Merkmale, Eigenschaften und Funktionen des in Figur 6 gezeigten bipolaren elektrochirurgischen Werkzeugs 20, in denen dieses sich von den anhand der Figuren 1 bis 5 dargestellten unterscheidet, beschrieben.

Das in Figur 6 gezeigte bipolare elektrochirurgische Werkzeug 20 unterscheidet sich von dem anhand der Figur 5 dargestellten Werkzeug insbesondere dadurch, dass nicht nur die zweite Greiffläche 41 an dem zweiten Maulteil 40 einen konvexen Bereich 42, sondern auch die erste Greiffläche 31 an dem ersten Maulteil 30 einen konvexen Bereich 32 aufweist. Der konvexe Bereich 32 der ersten Greiffläche 31 an dem ersten Maulteil 30 ist bei dem in Figur 6 dargestellten Beispiel durch einen Abschnitt mit trapezförmigem Querschnitt des elektrisch isolierenden zweiten Bauteils 35 des ersten Maulteils 30 gebildet. Bei dem dargestellten Beispiel sind die konvexen Bereiche 32, 42 der Greifflächen 31, 41 an den Maulteilen 30, 40 unterschiedlich hoch und unterschiedlich breit. Insbesondere ist ein dem ersten Maulteil 30 zugewandter ebener plateauförmiger Teilbereich 43 des konvexen Bereichs 42 der zweiten Greiffläche 41 an dem zweiten Maulteil 40 breiter als ein dem zweiten Maulteil 40 zugewandter ebener plateauförmiger Teilbereich 33 des konvexen Bereichs 32 der ersten Greiffläche 31 an dem ersten Maulteil 30.

Der konvexe Bereich 32 der ersten Greiffläche 31 an dem ersten Maulteil 30 ist von beiden Elektroden 61, 62 beabstandet. Zwischen den Maulteilen 30, 40 angeordnetes und zwischen den konvexen Bereichen 32, 42 komprimiertes Gewebe ist in der Umgebung der Elektrodenflächen 63, 64 nicht oder nur geringfügig komprimiert. Deshalb liegen in der Umgebung der Elektrodenflächen 63, 64 deutlich größere Querschnitte und deutlich kleinere Stromdichten vor als zwischen den konvexen Bereichen 32, 42. Damit ist die elektrochirurgische Wirkung auf das Gewebe im Wesentlichen auf den Bereich zwischen den konvexen Bereichen 32, 42 konzentriert.

Der plateauförmige Teilbereich 33 des konvexen Bereichs 32 der erste Greiffläche 31 und der plateauförmige Teilbereich 43 des konvexen Bereichs 42 der zweiten Greiffläche 41 sind jeweils eben oder im Wesentlichen eben und liegen einander parallel oder im Wesentlichen parallel gegenüber. Bei dem dargestellten Beispiel ist der plateauförmige Teilbereich 33 des konvexen Bereichs 32 der ersten Greiffläche 31 schmäler als der plateauförmige Teilbereich 43 des konvexen Bereichs 42 der zweiten Greiffläche 41. Die Breite des Versiegelungsbereichs oder Versiegelungsstreifens, innerhalb dessen das Gewebe versiegelt wird, entspricht im Wesentlichen der Breite des plateauförmigen Teilbereichs 33 des konvexen Bereichs 32 der ersten Greiffläche 31.

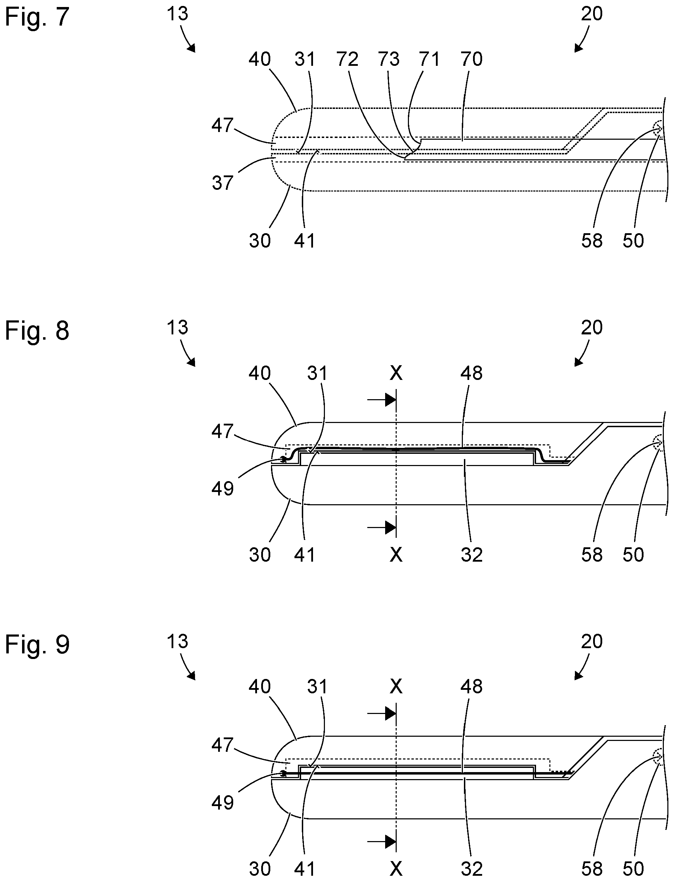

Figur 7 zeigt eine schematische Darstellung eines Werkzeugs 20 an einem distalen Ende 13 eines weiteren bipolaren elektrochirurgischen Instruments, das in einigen Merkmalen, Eigenschaften und Funktionen den anhand der Figuren 1 bis 6 dargestellten Instrumenten ähneln kann. Die Art der Darstellung in Figur 7 entspricht weitgehend derjenigen der Figuren 1, 2 und 4, ist jedoch gegenüber diesen vergrößert. Ferner sind die Maulteile 30, 40 in gestrichelten Linien und das Skalpell 70 in durchgezogenen Linien dargestellt, um die Eigenschaften des Skalpells 70 hervorzuheben. Nachfolgend sind insbesondere Merkmale, Eigenschaften und Funktionen des in Figur 7 gezeigten Werkzeugs 20, in denen dieses sich von den anhand der Figuren 1 bis 6 dargestellten Werkzeugen unterscheidet, beschrieben.

Das in Figur 7 gezeigte Werkzeug 20 unterscheidet sich von den anhand der Figuren 1 bis 6 dargestellten Werkzeugen insbesondere dadurch, dass die Schneide 71 des Skalpells 70 nicht gerade, sondern gebogen oder gekrümmt ist. Die Schneide 71 ist S-förmig und liegt insbesondere in einer Ebene parallel zur Zeichenebene der Figur 7. Die Schneide 71 weist an ihren Enden, die in den Nuten 37, 47 in den Maulteilen 30, 40 angeordnet und geführt sind, steile Abschnitte 72 auf, in denen die Schneide orthogonal oder fast orthogonal zu der vorgesehenen Bewegungsrichtung des Skalpells 70 ist. In einem mittleren Bereich weist die Schneide 71 einen flachen Abschnitt 73 auf, in dem die Schneide 71 in einem spitzen Winkel (bei dem dargestellten Beispiel: ca. 45 Grad) zu der vorgesehenen Bewegungsrichtung des Skalpells 70 verläuft. Wenn die Maulteile 30, 40 wie in Figur 7 angedeutet fast geschlossen sind oder einen geringen Abstand aufweisen, kann der flache Abschnitt 73 der Schneide 71 von den Maulteilen gehaltenes und gequetschtes Gewebe schneiden.

Abweichend von der Darstellung in Figur 7 kann die Schneide 71 nur an einem Ende einen steilen Abschnitt 72 aufweisen und/oder eine oder mehrere Ecken zwischen geraden oder gekrümmten Abschnitten aufweisen.

Die in Figur 7 gezeigte von der Geraden abweichende Gestalt der Schneide 71 kann bei einer kurzen Bauform der Schneide und damit einer Wirkung bis nahe zu dem distalen Ende 13 des Instruments und des Werkzeugs 20 gleichzeitig eine gute Führung des Skalpells in den Nuten 37, 47 und einen leichten Schnitt mittels des flachen Abschnitts 73 ermöglichen.

Figur 8 zeigt eine schematische Darstellung eines Werkzeugs 20 an einem distalen Ende 13 eines weiteren bipolaren elektrochirurgischen Instruments, das in einigen Merkmalen, Eigenschaften und Funktionen den anhand der Figuren 1 bis 7 dargestellten Instrumenten ähneln kann. Die Art der Darstellung in Figur 8 entspricht weitgehend derjenigen der Figur 7. Nachfolgend sind insbesondere Merkmale, Eigenschaften und Funktionen des in Figur 8 gezeigten Werkzeugs 20, in denen dieses sich von den anhand der Figuren 1 bis 7 dargestellten Werkzeugen unterscheidet, beschrieben.

Das in Figur 8 gezeigte Werkzeug 20 unterscheidet sich von den anhand der Figuren 1 bis 7 dargestellten Werkzeugen insbesondere dadurch, dass kein Skalpell zum mechanischen Durchtrennen von zwischen den Maulteilen 30, 40 gequetschtem und verschmolzenen, verschweißten oder versiegelten Gewebe vorgesehen ist. Stattdessen umfasst das Werkzeug 20 einen Schneiddraht 48 an dem zweiten Maulteil 40. Das distale Ende des Schneiddrahts 48 ist an einem distalen Befestigungspunkt 49 nahe dem distalen Ende des zweiten Maulteils zugfest befestigt. Bei der in Figur 8 gezeigten Konfiguration liegt der Schneiddraht 48 in einer Nut 47 in dem zweiten Maulteil 40.

Bei dem dargestellten Beispiel weist die erste Greiffläche 31 an dem ersten Maulteil 30 einen konvexen Bereich 32 auf, ähnlich wie bei dem anhand der Figur 6 dargestellten Werkzeug. Der konvexe Bereich 32 erstreckt sich nicht über die gesamte Länge des ersten Maulteils 30. Stattdessen ist das proximale Ende des konvexen Bereichs 32 von dem proximalen Ende der ersten Greiffläche 31 etwas beabstandet, und das distale Ende des konvexen Bereichs 32 ist von dem distalen Ende der ersten Greiffläche und dem distalen Ende des ersten Maulteils 30 etwas beabstandet.

Das zweite Maulteil 40 weist in Längsrichtung eine flache U-Form auf, die den konvexen Bereich 32 der ersten Greiffläche 31 umgreift. Bei dem dargestellten Beispiel sind der Längsschnitt des konvexen Bereichs 32 rechteckig und die flache U-Form des zweiten Maulteils 40 ebenfalls rechteckig. Die Nut 47 in dem zweiten Maulteil 40 ist deshalb in Längsrichtung auch nicht gerade, sondern flach rechteckig U-förmig. Der distale Befestigungspunkt 49 des Schneiddrahts 48 ist in der Nut 47 nahe deren distalem Ende angeordnet. Bei der in Figur 8 gezeigten geschlossenen Konfiguration liegt der distale Befestigungspunkt 49 des Schneiddrahts 48 distal des distalen Endes des konvexen Bereichs 32 der ersten Greiffläche 31.

Figur 9 zeigt eine weitere schematische Darstellung des Werkzeugs 20 aus Figur 8. Die Art der Darstellung in Figur 9 entspricht derjenigen der Figur 8.

Das Werkzeug 20 ist in Figur 9 in einer weiteren Konfiguration gezeigt, die von der in Figur 8 gezeigten Konfiguration ausgehend erreicht wird, indem das proximale Ende des Schneiddrahts 48 nach proximal gezogen wird. Dadurch wird der Schneiddraht 48 gestrafft, verlässt weitgehend die Nut 47 und nimmt eine gerade Gestalt an, die in Figur 9 gezeigt ist. In dieser geraden Gestalt liegt der Schneiddraht 48 in einer Längsnut in dem konvexen Bereich 32 der ersten Greiffläche 31 des ersten Maulteils 30.

Figur 10 zeigt eine schematische vergrößerte Darstellung eines Querschnitts entlang der in den Figuren 8 und 9 angedeuteten Ebene X-X orthogonal zu den Zeichenebenen der Figuren 8 und 9.

In diesem Querschnitt sind der konvexe Bereich 32 der ersten Greiffläche 31 des ersten Maulteils 30 und die Nuten 37, 47 in den Greifflächen 31, 41 erkennbar. Der Schneidraht 48 ist als schwarzer ausgefüllter Kreis in der anhand der Figur 8 dargestellten Konfiguration - also innerhalb der Nut 47 in der zweiten Greiffläche 41 - und als leerer Kreis in der anhand der Figur 9 dargestellten Konfiguration - also innerhalb der Nut 37 in der ersten Greiffläche 31 - gezeigt.

Während des Straffens des Schneiddrahts 48 und des Übergangs von der anhand der Figur 8 dargestellten Konfiguration zu der anhand der Figur 9 dargestellten Konfiguration, das heißt während der Bewegung des Schneiddrahts von der Nut 47 in der zweiten Greiffläche 41 in die Nut 37 in der ersten Greiffläche 31 kann der Schneiddraht als elektrochirurgisches Werkzeug Gewebe, das zwischen den Greifflächen 31, 41 der Maulteile 30, 40 gegriffen und gequetscht ist, elektrochirurgisch durchtrennen. Der Schneiddraht kann dabei als monopolares oder als bipolares elektrochirurgisches Werkzeug verwendet werden.

Figur 11 zeigt eine schematische Darstellung eines Querschnitts durch ein weiteres Werkzeug 20, das in einigen Merkmalen, Eigenschaften und Funktionen den anhand der Figuren 1 bis 10 dargestellten Werkzeugen ähneln kann. Die Art der Darstellung in Figur 11 entspricht weitgehend derjenigen der Figuren 3, 5, 6, 10. Nachfolgend sind insbesondere Merkmale, Eigenschaften und Funktionen des in Figur 11 gezeigten Werkzeugs 20, in denen dieses sich von den anhand der Figuren 1 bis 10 dargestellten Werkzeugen unterscheidet, beschrieben.

Das in Figur 11 gezeigte Werkzeug 20 unterscheidet sich von den anhand der Figuren 1 bis 10 dargestellten Werkzeugen insbesondere dadurch, dass die Greifflächen 31, 41 der Maulteile isolierend ausgebildet sind. Die von dem zweiten Maulteil 40 abgewandte Außenfläche des ersten Maulteils 30 ist als Elektrodenfläche 62 ausgebildet, und die von dem ersten Maulteil 30 abgewandte Außenfläche des zweiten Maulteils 40 ist als Elektrodenfläche 64 ausgebildet.

Bei dem dargestellten Beispiel umfasst das erste Maulteil 30 ein metallisches erste Bauteil 34, das gleichzeitig das erste Elektrodenbauteil 61 und die von dem zweiten Maulteil 40 abgewandte Außenfläche als Elektrodenfläche 63 bildet. Ferner umfasst das erste Maulteil 30 ein elektrisch isolierendes zweites Bauteil 35, das die gesamte erste Greiffläche 31 des ersten Maulteils 30 bildet. Das zweite Maulteil 40 umfasst ein metallisches erste Bauteil 44, das gleichzeitig das zweite Elektrodenbauteil 63 und die von dem ersten Maulteil 30 abgewandte Außenfläche als Elektrodenfläche 64 bildet. Ferner umfasst das zweite Maulteil 40 ein elektrisch isolierendes zweites Bauteil 45, das die gesamte zweite Greiffläche 41 des zweiten Maulteils 40 bildet.

Bei dem in Figur 11 dargestellten Beispiel sind Greifflächen 31, 41 der Maulteile 30, 40 dafür vorgesehen, bei der vorgesehenen Verwendung einen vorbestimmten Abstand aufzuweisen. Dieser Abstand kann beispielsweise durch einen in Figur 11 nicht dargestellten Anschlag formschlüssig definiert sein.

Figur 12 zeigt eine weitere schematische Darstellung eines Schnitts durch das anhand der Figur 11 dargestellte Werkzeug 20. Die Art der Darstellung von Figur 12 entspricht derjenigen der Figur 11.

In Figur 12 ist eine Situation gezeigt, in der Gewebe 90 zwischen den Maulteilen 30, 40 gegriffen und gequetscht ist. Das Werkzeug 20 ist mit dem gegriffenen und gequetschten Gewebe 90 um einen vorbestimmten Winkel rotiert, bei dem in Figur 12 gezeigten Beispiel um ca. 90 Grad. Dadurch liegt das Gewebe 90 außerhalb des gegriffenen und gequetschten Bereichs großflächig an den Elektrodenflächen 62, 64 an. In den Kontaktbereichen zwischen den Elektrodenflächen 62, 64 und dem Gewebe 90 und innerhalb des Gewebes außerhalb des gegriffenen und gequetschten Bereichs sind die Stromdichten aufgrund des großflächigen Kontakts relativ gering. Nur in dem gegriffenen und gequetschten Bereich ist die Stromdichte hoch, so dass nur dort oder im Wesentlich nur dort eine elektrochirurgische Wirkung eintritt.

### Bezugszeichen

- 10: bipolares elektrochirurgisches Instrument
- 11: Schaft des bipolaren elektrochirurgischen Instrument 10
- 12: distales Ende des Schafts 11
- 13: distales Ende des bipolaren elektrochirurgischen Instruments 10
- 20: bipolares elektrochirurgisches Werkzeug
- 30: erstes Maulteil oder erste Branche des Werkzeugs 20
- 31: erste Greiffläche an dem ersten Maulteil 30, dem zweiten Maulteil 40 zugewandt
- 32: konvexer Bereich der ersten Greiffläche 31
- 33: plateauförmiger Teilbereich des konvexen Bereichs 32
- 34: metallisches erstes Bauteil des ersten Maulteils 30
- 35: elektrisch isolierendes zweites Bauteil des ersten Maulteils 30
- 36: Außenfläche des ersten Maulteils 30, von dem zweiten Maulteil 40 abgewandt
- 37: Nut in der ersten Greiffläche 31
- 40: zweites Maulteil oder zweite Branche des Werkzeugs 20
- 41: zweite Greiffläche an dem zweiten Maulteil 40, dem ersten Maulteil 30 zugewandt
- 42: konvexer Bereich der zweiten Greiffläche 41
- 43: plateauförmiger Teilbereich des konvexen Bereichs 42
- 44: metallisches erstes Bauteil des zweiten Maulteil 40
- 45: elektrisch isolierendes zweites Bauteil des zweiten Maulteils 40
- 46: Außenfläche des zweiten Maulteils 40, von dem ersten Maulteil 30 abgewandt
- 47: Nut in der zweiten Greiffläche 41
- 48: Schneiddraht an dem zweiten Maulteil 40
- 49: distaler Befestigungspunkt des Schneiddrahts 48
- 50: Gelenk zwischen dem zweiten Maulteil 40 und dem ersten Maulteil 30
- 58: durch das Gelenk 50 definierte Schwenkachse des zweiten Maulteils 40
- 61: erstes Elektrodenbauteil in der ersten Greiffläche 31
- 62: zweites Elektrodenbauteil in der ersten Greiffläche 31
- 63: Elektrodenfläche an dem ersten Elektrodenbauteil 61
- 64: Elektrodenfläche an dem zweiten Elektrodenbauteil 62
- 70: Skalpell des Werkzeugs 20, in den Nuten 37, 47 bewegbar
- 71: Schneide des Skalpells 70
- 72: steiler Abschnitt der Schneide 71
- 73: flacher Abschnitt der Schneide 71
- 90: Gewebe

## Patentansprüche

1. **Bipolares elektrochirurgisches Werkzeug** (20) für ein bipolares elektrochirurgisches Instrument (10), mit:
einem **ersten Maulteil** (30);
einem **zweiten Maulteil** (40);
einem **Gelenk** (50), das eine Schwenkbewegung des zweiten Maulteils (40) relativ zu dem ersten Maulteil (30) ermöglicht;
einer **ersten Greiffläche** (31) an dem ersten Maulteil (30), die dem zweiten Maulteil (40) zugewandt ist;
einer von dem zweiten Maulteil (40) abgewandten **ersten Außenfläche** (36) an dem ersten Maulteil (30);
einer **zweiten Greiffläche** (41) an dem zweiten Maulteil (40), die dem ersten Maulteil (30) zugewandt ist;
einer von dem ersten Maulteil (30) abgewandten **zweiten Außenfläche** (46) an dem zweiten Maulteil (40);
einer **ersten Elektrode** (63), die **vollständig an der ersten Außenfläche** (36) des ersten Maulteils (30) angeordnet ist;
einer **zweiten Elektroden** (64), die von der ersten Elektrode (63) elektrisch isoliert und **vollständig an der zweiten Außenfläche** (46) des zweiten Maulteils (40) angeordnet ist,
**dadurch gekennzeichnet, dass**
die **erste Greiffläche** (31) an dem ersten Maulteil (30) vollständig **elektrisch isolierend** ausgebildet ist,
und dass die **zweite Greiffläche** (41) an dem zweiten Maulteil (40) vollständig **elektrisch isolierend** ausgebildet ist.

2. Bipolares elektrochirurgisches Werkzeug (20) nach einem der vorangehenden Ansprüche, bei dem
der gesamte dem ersten Maulteil zugewandte Oberflächenbereich des zweiten Maulteils elektrisch isolierend ausgebildet ist,
der gesamte dem zweiten Maulteil zugewandte Oberflächenbereich des ersten Maulteils elektrisch isolierend ausgebildet ist.

3. Bipolares elektrochirurgisches Werkzeug (20) nach einem der vorangehenden Ansprüche, bei dem das **erste Maulteil** (30) mit einem distalen Ende (12) eines **Schafts** (11) eines elektrochirurgischen Instruments (10) mechanisch starr verbindbar ist.

4. Bipolares elektrochirurgisches Werkzeug (20) nach einem der vorangehenden Ansprüche, bei dem die gesamte zweite Greiffläche (41) **elektrisch isolierend** ausgebildet ist.

5. Bipolares elektrochirurgisches Werkzeug (20) nach einem der vorangehenden Ansprüche, ferner mit:
einer Schneideinrichtung (70), die zwischen dem ersten Maulteil (30) und dem zweiten Maulteil (40) bewegbar ist, zum mechanischen Durchtrennen von zwischen dem ersten Maulteil (30) und dem zweiten Maulteil (40) gehaltenem Gewebe.

6. **Bipolares elektrochirurgisches Instrument** (10) mit:
einem **Schaft** (11);
einem **bipolaren elektrochirurgischen Werkzeug** (20) nach einem der vorangehenden Ansprüche,
wobei das bipolare elektrochirurgische Werkzeug (20) mit einem distalen Ende (12) des Schafts (11) verbunden oder verbindbar ist.

7. Bipolares elektrochirurgisches Instrument (10) nach Anspruch 6,
bei dem das erste Maulteil (30) mit dem distalen Ende (12) des Schafts (11) mechanisch starr verbunden oder starr verbindbar ist.

## Claims

1. A **bipolar electrosurgical tool** (20) for a bipolar electrosurgical instrument (10), comprising:
a **first jaw part** (30);
**a second jaw part** (40);
a **joint** (50) that enables the second jaw part (40) to move pivotally relative to the first jaw part (30);
a **first gripping face** (31) at the first jaw part (30) facing the second jaw part (40);
a **first outer face** (36) at the first jaw part (30) facing away from the second jaw part (40);
a **second gripping face** (41) at the second jaw part (40), facing the first jaw part (30);
a **second outer face** (46) at the second jaw part (40), facing away from the first jaw part (30);
a **first electrode** (63) arranged **entirely at the first outer face** (36) of the first jaw part (30);
a **second electrode** (64) which is electrically insulated from the first electrode (63) and arranged **entirely at the second outer face** (46) of the second jaw part (40), **characterised in that** the **first gripping face** (31) at the first jaw part (30) is **completely electrically insulating,**
and **in that** the **second gripping face** (41) at the second jaw part (40) is **completely electrically insulating.**

2. Bipolar electrosurgical instrument (20) according to one of the preceding claims, wherein
the entire surface area of the second jaw part facing the first jaw part is electrically insulating,
the entire surface area of the first jaw part facing the second jaw part is electrically insulating.

3. Bipolar electrosurgical tool (20) according to one of the preceding claims, wherein the **first jaw part** (30) can be mechanically rigidly connected to a distal end (12) of a shaft (11) of an electrosurgical instrument (10).

4. Bipolar electrosurgical instrument (20) according to one of the preceding claims, wherein the entire second gripping face (41) is **electrically insulating.**

5. Bipolar electrosurgical instrument (20) according to one of the preceding claims, further comprising:
a cutting device (70) that can be moved between the first jaw part (30) and the second jaw part (40), for mechanically cutting tissue held between the first jaw part (30) and the second jaw part (40).

6. **A bipolar electrosurgical instrument** (10) comprising:
a **shaft** (11);
a **bipolar electrosurgical tool** (20) according to one of the preceding claims,
wherein the bipolar electrosurgical tool (20) is connected to, or connectable to, a distal end (12) of the shaft (11).

7. Bipolar electrosurgical instrument (10) according to claim 6, wherein the first jaw part (30) is mechanically rigidly connected to, or can be rigidly connected to, the distal end (12) of the shaft (11).

## Revendications

1. **Outil électrochirurgical bipolaire** (20) pour un instrument électrochirurgical bipolaire (10), comprenant :
une **première partie de mâchoire** (30) ;
une **deuxième partie de mâchoire** (40) ;
une **articulation** (50) permettant un pivotement de la deuxième partie de mâchoire (40) par rapport à la première partie de mâchoire (30) ;
une **première surface de préhension** (31) au niveau de la première partie de mâchoire (30), qui est tournée vers la deuxième partie de mâchoire (40) ;
une **première surface extérieure** (36), détournée de la deuxième partie de mâchoire (40), au niveau de la première partie de mâchoire (30) ;
une **deuxième surface de préhension** (41) au niveau de la deuxième partie de mâchoire (40), qui est tournée vers la première partie de mâchoire (30) ;
une **deuxième surface extérieure** (46), détournée de la première partie de mâchoire (30), au niveau de la deuxième partie de mâchoire (40) ;
une **première électrode** (63) qui est disposée **entièrement** au niveau de la **première surface extérieure** (36) de la première partie de mâchoire (30) ;
une **deuxième électrode** (64) qui est électriquement isolée par rapport à la première électrode (63) et est disposée **entièrement** sur la **deuxième surface extérieure** (46) de la deuxième partie de mâchoire (40),
**caractérisé en ce que**
la **première surface de préhension** (31) est réalisée au niveau de la première partie de mâchoire (30) de manière entièrement **électriquement isolante,**
et **en ce que**
la **deuxième surface de préhension** (41) est réalisée au niveau de la deuxième partie de mâchoire (40) de manière entièrement **électriquement isolante.**

2. Outil électrochirurgical bipolaire (20) selon l'une quelconque des revendications précédentes, dans lequel
toute la zone superficielle de la deuxième partie de mâchoire, tournée vers la première partie de mâchoire, est réalisée de manière électriquement isolante,
toute la zone superficielle de la première partie de mâchoire, tournée vers la deuxième partie de mâchoire, est réalisée de manière électriquement isolante.

3. Outil électrochirurgical bipolaire (20) selon l'une quelconque des revendications précédentes, dans lequel la **première partie de mâchoire** (30) peut être reliée de manière mécaniquement rigide à une extrémité distale (12) d'une tige (11) d'un instrument électrochirurgical (10).

4. Outil électrochirurgical bipolaire (20) selon l'une quelconque des revendications précédentes, dans lequel toute la deuxième surface de préhension (41) est réalisée de manière **électriquement isolante.**

5. Outil électrochirurgical bipolaire (20) selon l'une quelconque des revendications précédentes, comprenant en outre :
un dispositif de coupe (70) qui est mobile entre la première partie de mâchoire (30) et la deuxième partie de mâchoire (40) pour sectionner mécaniquement des tissus maintenus entre la première partie de mâchoire (30) et la deuxième partie de mâchoire (40).

6. **Instrument électrochirurgical bipolaire** (10), comprenant :
une **tige** (11) ;
un **instrument électrochirurgical bipolaire** (20) selon l'une quelconque des revendications précédentes,
dans lequel l'outil électrochirurgical bipolaire (20) est ou peut être relié à une extrémité distale (12) du tige (11).

7. Instrument électrochirurgical bipolaire (10) selon la revendication 6, dans lequel la première partie de mâchoire (30) est ou peut être reliée de manière mécaniquement rigide à l'extrémité distale (12) du tige (11).
